# EUROPEAN PATENT SPECIFICATION

(11) **EP 3 548 011 B1**
(45) Date of publication and mention of the grant of the patent: **14.06.2023**
(21) Application number: 17835968.3
(22) Date of filing: 04.12.2017
(51) Int. Cl.: A61K 31/7088, A61K 31/7125, A61K 31/00, A61P 13/12, C12N 15/113

(54) **METHODS FOR TREATMENT OF POLYCYSTIC KIDNEY DISEASE**
VERFAHREN ZUR BEHANDLUNG VON POLYZYSTISCHER NIERENKRANKHEIT
PROCÉDÉS DE TRAITEMENT DE LA MALADIE POLYKYSTIQUE DES REINS

(30) Priority: 05.12.2016 US 201662430164 P
(43) Date of publication of application: 09.10.2019
(73) Proprietor: Regulus Therapeutics Inc., San Diego, CA 92121 (US); The Board of Regents of the University of Texas System, Austin, TX 78701 (US)
(72) Inventor: ALLERSON, Charles R., San Diego, California 92121 (US); PATEL, Vishal D., Austin, Texas 78701 (US); CHAU, B. Nelson, San Diego, California 92121 (US); ANDROSAVICH, John R., San Diego, California 92121 (US)
(74) Representative: Carpmaels & Ransford LLP
(86) International application number: PCT/US2017/064432
(87) International publication number: WO 2018/106568

(56) References cited:
- WO-A1-2009/109665
- WO-A1-2011/060100
- WO-A2-2005/103298
- WO-A2-2008/151639
- WO-A2-2015/123449
- HUAN SUN ET AL: "MicroRNA-17 post-transcriptionally regulates polycystic kidney disease-2 gene and promotes cell proliferation", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 37, no. 6, 10 October 2009 (2009-10-10), pages 2951-2958, XP019826622, ISSN: 1573-4978
- V. PATEL ET AL: "miR-17?92 miRNA cluster promotes kidney cyst growth in polycystic kidney disease", PROCEEDINGS NATIONAL ACADEMY OF SCIENCES PNAS, vol. 110, no. 26, 12 June 2013 (2013-06-12), pages 10765-10770, XP055320384, US ISSN: 0027-8424, DOI: 10.1073/pnas.1301693110
- ANDRIUS SERVA ET AL: "miR-17-5p Regulates Endocytic Trafficking through Targeting TBC1D2/Armus", PLOS ONE, vol. 7, no. 12, 1 January 2012 (2012-01-01), pages e52555-e52555, XP055111293, ISSN: 1932-6203, DOI: 10.1371/journal.pone.0052555

## Description

### FIELD OF INVENTION

Provided herein are compounds and pharmaceutical compositions for use in methods for the treatment of polycystic kidney disease.

### BACKGROUND

Polycystic kidney disease is characterized by the accumulation of numerous fluid-filled cysts in the kidney. These cysts are lined by a single layer of epithelial cells called the cyst epithelium. Over time, the cysts increase in size due to elevated cell proliferation and active secretion of fluid by the cyst epithelium. The enlarged cysts compress surrounding normal tissue, resulting in a decline of kidney function. The disease eventually progresses to end-stage renal disease, requiring dialysis or kidney transplant. At this stage, the cysts may be surrounded by areas of fibrosis containing atrophic tubules.

A number of genetic disorders can result in polycystic kidney disease (PKD). The various forms of PKD are distinguished by the manner of inheritance, for example, autosomal dominant or autosomal recessive inheritance; the involvement of organs and presentation of phenotypes outside of the kidney; the age of onset of end-stage renal disease, for example, at birth, in childhood or adulthood; and the underlying genetic mutation that is associated with the disease. *See,* for example, Kurschat et al., 2014, Nature Reviews Nephrology, 10: 687-699.

*See,* also, Sun et al., 2009, Molecular Biology Reports, vol. 37, no. 6, pages 2951-2958; Patel et al., 2013, Proceedings National Academy of Sciences (PNAS), vol. 110, no. 26, pages 10765-10770; Serva et al., 2012, PLOS One, vol. 7, no. 12, pages e52555-e52555; WO 2015/123449 A2 (Univ Jefferson), 20 August 2015; WO 2011/060100 A1 (Sanford Burnham Med Res Inst; Rana Tariq M), 19 May 2011.

### SUMMARY OF INVENTION

The invention provides a compound for use in a method of treating polycystic kidney disease, wherein the method comprises administering the compound to a subject in need thereof, and wherein the compound comprises a modified oligonucleotide consisting of 9 linked nucleosides, wherein the modified oligonucleotide has the following nucleoside pattern in the 5' to 3' orientation:
N_{S}N_{S}N_{M}N_{F}N_{F}N_{F}N_{M}N_{S}N_{S}
wherein nucleosides followed by subscript "M" are 2'-O-methyl nucleosides, nucleosides followed by subscript "F" are 2'-fluoro nucleosides, nucleosides followed by subscript "S" are S-cEt nucleosides, and all linkages are phosphorothioate linkages; and
wherein the nucleobase sequence of the modified oligonucleotide is 5'-AGCACUUUG-3', wherein each cytosine is independently selected from a non-methylated cytosine and a 5-methylcytosine; or a pharmaceutically acceptable salt thereof.

The invention also provides a pharmaceutical composition for use in a method of treating polycystic kidney disease, wherein the method comprises administering the pharmaceutical composition to a subject in need thereof, and wherein the pharmaceutical composition comprises:
a) a compound comprising a modified oligonucleotide consisting of 9 linked nucleosides, wherein the modified oligonucleotide has the following nucleoside pattern in the 5' to 3' orientation:
   N_{S}N_{S}N_{M}N_{F}N_{F}N_{F}N_{M}N_{S}N_{S}
   wherein nucleosides followed by subscript "M" are 2'-O-methyl nucleosides, nucleosides followed by subscript "F" are 2'-fluoro nucleosides, nucleosides followed by subscript "S" are S-cEt nucleosides, and all linkages are phosphorothioate linkages; and wherein the nucleobase sequence of the modified oligonucleotide is 5'-AGCACUUUG-3', wherein each cytosine is independently selected from a non-methylated cytosine and a 5-methylcytosine; or a pharmaceutically acceptable salt thereof; and
b) a pharmaceutically acceptable diluent.

Other embodiments of the invention are set out in the claims. The scope of the invention is defined by the appended claims.

Further embodiments are set out below.

Embodiment 1. In some embodiments, the subject, prior to administration of the compound or pharmaceutical composition, was determined to have an increased level of miR-17 in the kidney, urine or blood of the subject.

Embodiment 2. In some embodiments, the subject has a mutation selected from a mutation in the *PKD1* gene or a mutation in the *PKD2* gene.

Embodiment 3. In some embodiments, the subject has increased total kidney volume. Embodiment 4. In some embodiments, the subject has hypertension.

Embodiment 5. In some embodiments, the subject has impaired kidney function.

Embodiment 6. In some embodiments, the subject is in need of improved kidney function. Embodiment 7. In some embodiments, the method comprises:
a) measuring total kidney volume in the subject;
b) measuring hypertension in the subject;
c) measuring kidney pain in the subject;
d) measuring fibrosis in the kidney of the subject;
e) measuring blood urea nitrogen level in the subject;
f) measuring serum creatinine level in the subject;
g) measuring creatinine clearance in the subject;
h) measuring albuminuria in the subject;
i) measuring albumin:creatinine ratio in the subject;
j) measuring glomerular filtration rate in the subject;
k) measuring neutrophil gelatinase-associated lipocalin (NGAL) protein in the urine of the subject; and/or
l) measuring kidney injury molecule-1 (KIM-1) protein in the urine of the subject.

Embodiment 8. In some embodiments, the administering reduces total kidney volume in the subject.

Embodiment 9. In some embodiments, the administering slows the rate of increase of total kidney volume in the subject.

Embodiment 10. In some embodiments, the total kidney volume is height-adjusted total kidney volume.

Embodiment 11. In some embodiments, the administering slows the rate of decline of glomerular filtration rate in the subject.

Embodiment 12. In some embodiments, the glomerular filtration rate is estimated glomerular filtration rate.

Embodiment 13. In some embodiments, the cyst is present in one or more kidneys in the subject.

Embodiment 14. In some embodiments, the cyst is present in the liver of the subject.

Embodiment 15. In some embodiments, the method comprises administering at least one additional therapy, wherein at least one additional therapy is an anti-hypertensive agent.

In some embodiments, the subject is a human subject.

In some embodiments, each cytosine is a non-methylated cytosine.

### BRIEF DESCRIPTION OF FIGURES

**Figure 1A-1B****.** (A) Activity of RG4326 in miR-17 luciferase assay. (B) Activity RG4326 in miR-17 family member luciferase assays.
**Figure 2****.** PD signature score in IMCD3 cells following treatment with RG4326 or control RG5124.
**Figure 3A-3B****.** miPSA showing miR-17 target engagement in (A) kidney of wild-type mice and (B) kidney of RG4326-treated mice.
**Figure 4A-4C****.** Efficacy of RG4326 in the *Pkd2-KO* model of PKD. Effects of treatment on (A) kidney-to-body weight ratio, (B) blood urea nitrogen (BUN) level and (C) cystic index.
**Figure 5A-5C****.** Efficacy of RG4326 in the Pcy model of PKD. Effects of treatment on (A) kidney-to-body weight ratio, (B) blood urea nitrogen (BUN) level and (C) cystic index.

### DETAILED DESCRIPTION

Unless defined otherwise, all technical and scientific terms used herein have the same meaning as is commonly understood by one of skill in the arts to which the invention belongs. Unless specific definitions are provided, the nomenclature utilized in connection with, and the procedures and techniques of, analytical chemistry, synthetic organic chemistry, and medicinal and pharmaceutical chemistry described herein are those well-known and commonly used in the art. In the event that there is a plurality of definitions for terms herein, those in this section prevail. Standard techniques may be used for chemical synthesis, chemical analysis, pharmaceutical preparation, formulation and delivery, and treatment of subjects. Certain such techniques and procedures may be found for example in "Carbohydrate Modifications in Antisense Research" Edited by Sanghvi and Cook, American Chemical Society, Washington D.C., 1994; and "Remington's Pharmaceutical Sciences," Mack Publishing Co., Easton, Pa., 18th edition, 1990. Where reference is made to a URL or other such identifier or address, it is understood that such identifiers can change and particular information on the internet can change, but equivalent information can be found by searching the internet. Reference thereto evidences the availability and public dissemination of such information.

Before the present compositions and methods are disclosed and described, it is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise.

### Definitions

"Polycystic kidney disease" or "PKD" is a cystic kidney disease characterized by the accumulation of numerous fluid-filled cysts in the kidney. Multiple cysts form in at least one kidney, frequently leading to enlargement of the affected kidney(s) and progressive loss of kidney function.

"Marker of polycystic kidney disease" means a medical parameter that is used to assess severity of polycystic kidney disease, kidney function, and/or response of a subject having polycystic kidney disease to treatment. Non-limiting examples of markers of polycystic kidney disease include total kidney volume, hypertension, glomerular filtration rate, and kidney pain.

"Marker of kidney function" means a medical parameter that is used to assess kidney function in a subject. Non-limiting examples of markers of kidney function include glomerular filtration rate, blood urea nitrogen level, and serum creatinine level.

"Autosomal dominant polycystic kidney disease" or "ADPKD" is a polycystic kidney disease caused by one or more genetic mutations in the *PKD1* and/or *PKD2* gene. 85% of ADPKD is caused by mutations in *PKD1,* which is located on chromosome 16, with the majority of the remaining ADPKD cases caused by mutations in *PKD2,* which is located on chromosome 4.

"Autosomal recessive polycystic kidney disease" or "ARPKD" is a polycystic kidney disease caused by one or more genetic mutations in the *PKHD1* gene, which is located on chromosome 6. Up to 50% of neonates with ARPKD die from complications of intrauterine kidney disease, and about a third of those who survive develop end stage renal disease (ESRD) within 10 years.

"Nephronophthisis" or "NPHP" means an autosomal recessive cystic kidney disease characterized by corticomedullary cysts, tubular basement membrane disruption, and tubulointerstitial nephropathy.

"Total kidney volume" or "TKV" is a measurement of total kidney volume. Total kidney volume may be determined by Magnetic Resonance Imaging (MRI), Computed Tomography (CT) scan, or ultrasound (US) imaging, and the volume calculated by a standard methodology, such as an ellipsoid volume equation (for ultrasound), or by quantitative stereology or boundary tracing (for CT/MRI).

"Height-adjusted total kidney volume" or "HtTKV" is a measure of total kidney volume per unit height. Patients with an HtTKV value ≥ 600 ml/m are predicted to develop stage 3 chronic kidney disease within 8 years.

"Kidney pain" means clinically significant kidney pain necessitating medical leave, pharmacologic treatment (narcotic or last-resort analgesic agents), or invasive intervention.

"Worsening hypertension" means a change in blood pressure that requires initiation of or an increase in hypertensive treatment.

"Fibrosis" means the formation or development of excess fibrous connective tissue in an organ or tissue. In certain embodiments, fibrosis occurs as a reparative or reactive process. In certain embodiments, fibrosis occurs in response to damage or injury. The term "fibrosis" is to be understood as the formation or development of excess fibrous connective tissue in an organ or tissue as a reparative or reactive process, as opposed to a formation of fibrous tissue as a normal constituent of an organ or tissue.

"Hematuria" means the presence of red blood cells in the urine.

"Albuminuria" means the presence of excess albumin in the urine, and includes without limitation, normal albuminuria, high normal albuminuria, microalbuminuria and macroalbuminuria. Normally, the glomerular filtration permeability barrier, which is composed of podocyte, glomerular basement membrane and endothelial cells, prevents serum protein from leaking into urine. Albuminuria may reflect injury of the glomerular filtration permeability barrier. Albuminuria may be calculated from a 24-hour urine sample, an overnight urine sample or a spot-urine sample.

"High normal albuminuria" means elevated albuminuria characterized by (i) the excretion of 15 to <30 mg of albumin into the urine per 24 hours and/or (ii) an albumin/creatinine ratio of 1.25 to <2.5 mg/mmol (or 10 to <20 mg/g) in males or 1.75 to <3.5 mg/mmol (or 15 to <30 mg/g) in females.

"Microalbuminuria" means elevated albuminuria characterized by (i) the excretion of 30 to 300 mg of albumin into the urine per 24 hours and/or (ii) an albumin/creatinine ratio of 2.5 to <25 mg/mmol (or 20 to <200 mg/g) in males or 3.5 to <35 mg/mmol (or 30 to <300 mg/g) in females.

"Macroalbuminuria" means elevated albuminuria characterized by the excretion of more than 300 mg of albumin into the urine per 24 hours and/or (ii) an albumin/creatinine ratio of >25 mg/mmol (or >200 mg/g) in males or >35 mg/mmol (or >300 mg/g) in females.

"Albumin/creatinine ratio" means the ratio of urine albumin (mg/dL) per urine creatinine (g/dL) and is expressed as mg/g. In certain embodiments, albumin/creatinine ratio may be calculated from a spot-urine sample and may be used as an estimate of albumin excretion over a 24-hour period.

"Glomerular filtration rate" or "GFR" means the flow rate of filtered fluid through the kidney and is used as an indicator of kidney function in a subject. In certain embodiments, a subject's GFR is determined by calculating an estimated glomerular filtration rate. In certain embodiments, a subject's GFR is directly measured in the subject, using the inulin method.

"Estimated glomerular filtration rate" or "eGFR" means a measurement of how well the kidneys are filtering creatinine, and is used to approximate glomerular filtration rate. As the direct measurement of GFR is complex, eGFR is frequently used in clinical practice. Normal results may range from 90-120 mL/min/1.73 m². Levels below 60 mL/min/1.73 m² for 3 or more months may be an indicator chronic kidney disease. Levels below 15 mL/min/1.73 m² may be an indicator of kidney failure.

"Proteinuria" means the presence of an excess of serum proteins in the urine. Proteinuria may be characterized by the excretion of > 250 mg of protein into the urine per 24 hours and/or a urine protein to creatinine ratio of ≥ 0.20 mg/mg. Serum proteins elevated in association with proteinuria include, without limitation, albumin.

"Blood urea nitrogen level" or "BLTN level" means a measure of the amount of nitrogen in the blood in the form of urea. The liver produces urea in the urea cycle as a waste product of the digestion of protein, and the urea is removed from the blood by the kidneys. Normal human adult blood may contain between 7 to 21 mg of urea nitrogen per 100 ml (7-21 mg/dL) of blood. Measurement of blood urea nitrogen level is used as an indicator of renal health. If the kidneys are not able to remove urea from the blood normally, a subject's BUN level rises.

"Elevated" means an increase in a medical parameter that is considered clinically relevant. A health professional may determine whether an increase is clinically significant.

"End stage renal disease (ESRD)" means the complete or almost complete failure of kidney function.

"Quality of life" means the extent to which a subject's physical, psychological, and social functioning are impaired by a disease and/or treatment of a disease. Quality of life may be reduced in subjects having polycystic kidney disease.

"Impaired kidney function" means reduced kidney function, relative to normal kidney function.

"Slow the worsening of" and "slow worsening" mean to reduce the rate at which a medical condition moves towards an advanced state.

"Delay time to dialysis" means to maintain sufficient kidney function such that the need for dialysis treatment is delayed.

"Delay time to renal transplant" means to maintain sufficient kidney function such that the need for a kidney transplant is delayed.

"Improves life expectancy" means to lengthen the life of a subject by treating one or more symptoms of a disease in the subject.

"Subject" means a human or non-human animal selected for treatment or therapy.

"Subject in need thereof' means a subject that is identified as in need of a therapy or treatment.

"Subject suspected of having" means a subject exhibiting one or more clinical indicators of a disease.

"Disease associated with miR-17" means a disease or condition that is modulated by the activity of one or more miR-17 family members.

"Administering" means providing a pharmaceutical agent or composition to a subject, and includes, but is not limited to, administering by a medical professional and self-administering.

"Parenteral administration" means administration through injection or infusion.
Parenteral administration includes, but is not limited to, subcutaneous administration, intravenous administration, and intramuscular administration.

"Subcutaneous administration" means administration just below the skin.

"Intravenous administration" means administration into a vein.

"Administered concomitantly" refers to the co-administration of two or more agents in any manner in which the pharmacological effects of both are manifest in the patient at the same time. Concomitant administration does not require that both agents be administered in a single pharmaceutical composition, in the same dosage form, or by the same route of administration. The effects of both agents need not manifest themselves at the same time. The effects need only be overlapping for a period and need not be coextensive.

"Duration" means the period during which an activity or event continues. In certain embodiments, the duration of treatment is the period during which doses of a pharmaceutical agent or pharmaceutical composition are administered.

"Therapy" means a disease treatment method. In certain embodiments, therapy includes, but is not limited to, administration of one or more pharmaceutical agents to a subject having a disease.

"Treat" means to apply one or more specific procedures used for the amelioration of at least one indicator of a disease. In certain embodiments, the specific procedure is the administration of one or more pharmaceutical agents. In certain embodiments, treatment of PKD includes, but is not limited to, reducing total kidney volume, improving kidney function, reducing hypertension, and/or reducing kidney pain.

"Ameliorate" means to lessen the severity of at least one indicator of a condition or disease. In certain embodiments, amelioration includes a delay or slowing in the progression of one or more indicators of a condition or disease. The severity of indicators may be determined by subjective or objective measures which are known to those skilled in the art.

"At risk for developing" means the state in which a subject is predisposed to developing a condition or disease. In certain embodiments, a subject at risk for developing a condition or disease exhibits one or more symptoms of the condition or disease, but does not exhibit a sufficient number of symptoms to be diagnosed with the condition or disease. In certain embodiments, a subject at risk for developing a condition or disease exhibits one or more symptoms of the condition or disease, but to a lesser extent required to be diagnosed with the condition or disease.

"Prevent the onset of" means to prevent the development of a condition or disease in a subject who is at risk for developing the disease or condition. In certain embodiments, a subject at risk for developing the disease or condition receives treatment similar to the treatment received by a subject who already has the disease or condition.

"Delay the onset of" means to delay the development of a condition or disease in a subject who is at risk for developing the disease or condition. In certain embodiments, a subject at risk for developing the disease or condition receives treatment similar to the treatment received by a subject who already has the disease or condition.

"Dose" means a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual. In certain embodiments, a dose is administered as a slow infusion.

"Dosage unit" means a form in which a pharmaceutical agent is provided. In certain embodiments, a dosage unit is a vial containing lyophilized oligonucleotide. In certain embodiments, a dosage unit is a vial containing reconstituted oligonucleotide.

"Therapeutically effective amount" refers to an amount of a pharmaceutical agent that provides a therapeutic benefit to an animal.

"Pharmaceutical composition" means a mixture of substances suitable for administering to an individual that includes a pharmaceutical agent. For example, a pharmaceutical composition may comprise a sterile aqueous solution.

"Pharmaceutical agent" means a substance that provides a therapeutic effect when administered to a subject.

"Active pharmaceutical ingredient" means the substance in a pharmaceutical composition that provides a desired effect.

"Pharmaceutically acceptable salt" means a physiologically and pharmaceutically acceptable salt of a compound provided herein, *i.e.,* a salt that retains the desired biological activity of the compound and does not have undesired toxicological effects when administered to a subject. Nonlimiting exemplary pharmaceutically acceptable salts of compounds provided herein include sodium and potassium salt forms. The terms "compound," "oligonucleotide," and "modified oligonucleotide" as used herein include pharmaceutically acceptable salts thereof unless specifically indicated otherwise.

"Saline solution" means a solution of sodium chloride in water.

"Improved organ function" means a change in organ function toward normal limits. In certain embodiments, organ function is assessed by measuring molecules found in a subject's blood or urine. For example, in certain embodiments, improved kidney function is measured by a reduction in blood urea nitrogen level, a reduction in proteinuria, a reduction in albuminuria, etc.

"Acceptable safety profile" means a pattern of side effects that is within clinically acceptable limits.

"Side effect" means a physiological response attributable to a treatment other than desired effects. In certain embodiments, side effects include, without limitation, injection site reactions, liver function test abnormalities, kidney function abnormalities, liver toxicity, renal toxicity, central nervous system abnormalities, and myopathies. Such side effects may be detected directly or indirectly. For example, increased aminotransferase levels in serum may indicate liver toxicity or liver function abnormality. For example, increased bilirubin may indicate liver toxicity or liver function abnormality.

The term "blood" as used herein, encompasses whole blood and blood fractions, such as serum and plasma.

"Anti-miR" means an oligonucleotide having a nucleobase sequence complementary to a microRNA. An anti-miR may be a modified oligonucleotide.

"Anti-miR-17" means a modified oligonucleotide having a nucleobase sequence complementary to one or more miR-17 family members. For example, an anti-miR-17 may be fully complementary (i.e., 100% complementary) to one or more miR-17 family members. For example, an anti-miR-17 may be at least 80%, at least 85%, at least 90%, or at least 95% complementary to one or more miR-17 family members.

"miR-17" means the mature miRNA having the nucleobase sequence 5'-CAAAGUGCUUACAGUGCAGGUAG-3' (SEQ ID NO: 1).

"miR-20a" means the mature miRNA having the nucleobase sequence 5'-UAAAGUGCUUAUAGUGCAGGUAG-3' (SEQ ID NO: 2).

"miR-20b" means the mature miRNA having the nucleobase sequence 5'-CAAAGUGCUCAUAGUGCAGGUAG -3' (SEQ ID NO: 3).

"miR-93" means the mature miRNA having the nucleobase sequence 5'-CAAAGUGCUGUUCGUGCAGGUAG-3' (SEQ ID NO: 4).

"miR-106a" means the mature miRNA having the nucleobase sequence 5'-AAAAGUGCUUACAGUGCAGGUAG-3' (SEQ ID NO: 5).

"miR-106b" means the mature miRNA having the nucleobase sequence 5'-UAAAGUGCUGACAGUGCAGAU-3' (SEQ ID NO: 6).

"miR-17 seed sequence" means the nucleobase sequence 5'-AAAGUG-3,' which is present in each of the miR-17 family members.

"miR-17 family member" means a mature miRNA having a nucleobase sequence comprising the miR-17 seed sequence, and which is selected from miR-17, miR-20a, miR-20b, miR-93, miR-106a, and miR-106b.

"miR-17 family" means the following group of miRNAs: miR-17, miR-20a, miR-20b, miR-93, miR-106a, and miR-106b, each having a nucleobase sequence comprising the miR-17 seed sequence.

"Target nucleic acid" means a nucleic acid to which an oligomeric compound is designed to hybridize.

"Targeting" means the process of design and selection of nucleobase sequence that will hybridize to a target nucleic acid.

"Targeted to" means having a nucleobase sequence that will allow hybridization to a target nucleic acid.

"Modulation" means a perturbation of function, amount, or activity. In certain embodiments, modulation means an increase in function, amount, or activity. In certain embodiments, modulation means a decrease in function, amount, or activity.

"Expression" means any functions and steps by which a gene's coded information is converted into structures present and operating in a cell.

"Nucleobase sequence" means the order of contiguous nucleobases in an oligomeric compound or nucleic acid, typically listed in a 5' to 3' orientation, and independent of any sugar, linkage, and/or nucleobase modification.

"Contiguous nucleobases" means nucleobases immediately adjacent to each other in a nucleic acid.

"Nucleobase complementarity" means the ability of two nucleobases to pair non-covalently via hydrogen bonding.

"Complementary" means that one nucleic acid is capable of hybridizing to another nucleic acid or oligonucleotide. In certain embodiments, complementary refers to an oligonucleotide capable of hybridizing to a target nucleic acid.

"Fully complementary" means each nucleobase of an oligonucleotide is capable of pairing with a nucleobase at each corresponding position in a target nucleic acid. In certain embodiments, an oligonucleotide is fully complementary (also referred to as 100% complementary) to a microRNA, i.e. each nucleobase of the oligonucleotide is complementary to a nucleobase at a corresponding position in the microRNA. A modified oligonucleotide may be fully complementary to a microRNA, and have a number of linked nucleosides that is less than the length of the microRNA. For example, an oligonucleotide with 16 linked nucleosides, where each nucleobase of the oligonucleotide is complementary to a nucleobase at a corresponding position in a microRNA, is fully complementary to the microRNA. In certain embodiments, an oligonucleotide wherein each nucleobase has complementarity to a nucleobase within a region of a microRNA stem-loop sequence is fully complementary to the microRNA stem-loop sequence.

"Percent complementarity" means the percentage of nucleobases of an oligonucleotide that are complementary to an equal-length portion of a target nucleic acid. Percent complementarity is calculated by dividing the number of nucleobases of the oligonucleotide that are complementary to nucleobases at corresponding positions in the target nucleic acid by the total number of nucleobases in the oligonucleotide.

"Percent identity" means the number of nucleobases in a first nucleic acid that are identical to nucleobases at corresponding positions in a second nucleic acid, divided by the total number of nucleobases in the first nucleic acid. In certain embodiments, the first nucleic acid is a microRNA and the second nucleic acid is a microRNA. In certain embodiments, the first nucleic acid is an oligonucleotide and the second nucleic acid is an oligonucleotide.

"Hybridize" means the annealing of complementary nucleic acids that occurs through nucleobase complementarity.

"Mismatch" means a nucleobase of a first nucleic acid that is not capable of Watson-Crick pairing with a nucleobase at a corresponding position of a second nucleic acid.

"Identical" in the context of nucleobase sequences, means having the same nucleobase sequence, independent of sugar, linkage, and/or nucleobase modifications and independent of the methylation state of any pyrimidines present.

"MicroRNA" means an endogenous non-coding RNA between 18 and 25 nucleobases in length, which is the product of cleavage of a pre-microRNA by the enzyme Dicer. Examples of mature microRNAs are found in the microRNA database known as miRBase (microrna.sanger.ac.ukI). In certain embodiments, microRNA is abbreviated as "miR."

"microRNA-regulated transcript" means a transcript that is regulated by a microRNA.

"Seed match sequence" means a nucleobase sequence that is complementary to a seed sequence, and is the same length as the seed sequence.

"Oligomeric compound" means a compound that comprises a plurality of linked monomeric subunits. Oligomeric compounds include oligonucleotides.

"Oligonucleotide" means a compound comprising a plurality of linked nucleosides, each of which can be modified or unmodified, independent from one another.

"Naturally occurring internucleoside linkage" means a 3' to 5' phosphodiester linkage between nucleosides.

"Natural sugar" means a sugar found in DNA (2'-H) or RNA (2'-OH).

"Internucleoside linkage" means a covalent linkage between adjacent nucleosides.

"Linked nucleosides" means nucleosides joined by a covalent linkage.

"Nucleobase" means a heterocyclic moiety capable of non-covalently pairing with another nucleobase.

"Nucleoside" means a nucleobase linked to a sugar moiety.

"Nucleotide" means a nucleoside having a phosphate group covalently linked to the sugar portion of a nucleoside.

"Compound comprising a modified oligonucleotide consisting of" a number of linked nucleosides means a compound that includes a modified oligonucleotide having the specified number of linked nucleosides. Thus, the compound may include additional substituents or conjugates. Unless otherwise indicated, the modified oligonucleotide is not hybridized to a complementary strand and the compound does not include any additional nucleosides beyond those of the modified oligonucleotide.

"Modified oligonucleotide" means a single-stranded oligonucleotide having one or more modifications relative to a naturally occurring terminus, sugar, nucleobase, and/or internucleoside linkage. A modified oligonucleotide may comprise unmodified nucleosides.

"Modified nucleoside" means a nucleoside having any change from a naturally occurring nucleoside. A modified nucleoside may have a modified sugar and an unmodified nucleobase. A modified nucleoside may have a modified sugar and a modified nucleobase. A modified nucleoside may have a natural sugar and a modified nucleobase. In certain embodiments, a modified nucleoside is a bicyclic nucleoside. In certain embodiments, a modified nucleoside is a non-bicyclic nucleoside.

"Modified internucleoside linkage" means any change from a naturally occurring internucleoside linkage.

"Phosphorothioate internucleoside linkage" means a linkage between nucleosides where one of the non-bridging atoms is a sulfur atom.

"Modified sugar moiety" means substitution and/or any change from a natural sugar.

"Unmodified nucleobase" means the naturally occurring heterocyclic bases of RNA or DNA: the purine bases adenine (A) and guanine (G), and the pyrimidine bases thymine (T), cytosine (C) (including 5-methylcytosine), and uracil (U).

"5-methylcytosine" means a cytosine comprising a methyl group attached to the 5 position.

"Non-methylated cytosine" means a cytosine that does not have a methyl group attached to the 5 position.

"Modified nucleobase" means any nucleobase that is not an unmodified nucleobase.

"Sugar moiety" means a naturally occurring furanosyl or a modified sugar moiety.

"Modified sugar moiety" means a substituted sugar moiety or a sugar surrogate.

"2'-O-methyl sugar" or "2'-OMe sugar" means a sugar having an O-methyl modification at the 2' position.

"2'-O-methoxyethyl sugar" or "2'-MOE sugar" means a sugar having an O-methoxyethyl modification at the 2' position.

"2'-fluoro" or "2'-F" means a sugar having a fluoro modification of the 2' position.

"Bicyclic sugar moiety" means a modified sugar moiety comprising a 4 to 7 membered ring (including by not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments, the 4 to 7 membered ring is a furanosyl. In certain such embodiments, the bridge connects the 2'-carbon and the 4'-carbon of the furanosyl. Nonlimiting exemplary bicyclic sugar moieties include LNA, ENA, cEt, S-cEt, and R-cEt.

"Locked nucleic acid (LNA) sugar moiety" means a substituted sugar moiety comprising a (CH₂)-O bridge between the 4' and 2' furanose ring atoms.

"ENA sugar moiety" means a substituted sugar moiety comprising a (CH₂)₂-O bridge between the 4' and 2' furanose ring atoms.

"Constrained ethyl (cEt) sugar moiety" means a substituted sugar moiety comprising a CH(CH₃)-O bridge between the 4' and the 2' furanose ring atoms. In certain embodiments, the CH(CH₃)-O bridge is constrained in the S orientation. In certain embodiments, the CH(CH₃)-O is constrained in the R orientation.

"S-cEt sugar moiety" means a substituted sugar moiety comprising an S-constrained CH(CH₃)-O bridge between the 4' and the 2' furanose ring atoms.

"R-cEt sugar moiety" means a substituted sugar moiety comprising an R-constrained CH(CH₃)-O bridge between the 4' and the 2' furanose ring atoms.

"2'-O-methyl nucleoside" means a 2'-modified nucleoside having a 2'-O-methyl sugar modification.

"2'-O-methoxyethyl nucleoside" means a 2'-modified nucleoside having a 2'-O-methoxyethyl sugar modification. A 2'-O-methoxyethyl nucleoside may comprise a modified or unmodified nucleobase.

"2'-fluoro nucleoside" means a 2'-modified nucleoside having a 2'-fluoro sugar modification. A 2'-fluoro nucleoside may comprise a modified or unmodified nucleobase.

"Bicyclic nucleoside" means a 2'-modified nucleoside having a bicyclic sugar moiety. A bicyclic nucleoside may have a modified or unmodified nucleobase.

"cEt nucleoside" means a nucleoside comprising a cEt sugar moiety. A cEt nucleoside may comprise a modified or unmodified nucleobase.

"S-cEt nucleoside" means a nucleoside comprising an S-cEt sugar moiety.

"R-cEt nucleoside" means a nucleoside comprising an R-cEt sugar moiety.

"β-D-deoxyribonucleoside" means a naturally occurring DNA nucleoside.

"β-D-ribonucleoside" means a naturally occurring RNA nucleoside.

"LNA nucleoside" means a nucleoside comprising a LNA sugar moiety.

"ENA nucleoside" means a nucleoside comprising an ENA sugar moiety.

### Overview

Polycystic kidney disease (PKD) is an inherited form of kidney disease in which fluid-filled cysts develop in the kidneys, leading torenal insufficiency, and often end-stage renal disease. Certain PKDs are also characterized by kidney enlargement. The excessive proliferation of cysts is a hallmark pathological feature of PKD. In the management of PKD, the primary goal for treatment is to manage symptoms such as hypertension and infections, maintain kidney function and prevent the onset of end-stage renal disease (ESRD), which in turn improves life expectancy of subjects with PKD.

miR-17 family members of the miR-17∼92 cluster of microRNAs are upregulated in mouse models of PKD. Genetic deletion of the miR-17∼92 cluster in a mouse model of PKD reduces kidney cyst growth, improves renal function, and prolongs survival (Patel et al., PNAS, 2013; 110(26): 10765-10770). Inhibition of miR-17 with a research tool compound has been shown to reduce kidney-to-body weight ratio and improve kidney function in an experimental model of PKD. Further, miR-17 inhibition also suppressed proliferation and cyst growth of primary cultures derived from cysts of human donors.

To identify inhibitors of one or more miR-17 family members that are sufficiently efficacious, safe and convenient to administer to subjects with PKD, approximately 200 modified oligonucleotides comprising a nucleobase sequence complementary to the miR-17 seed sequence were designed, having varying lengths and chemical composition. The length of the compounds ranged from 9 to 20 linked nucleosides, and the compounds varied in the number, type, and placement of chemical modifications. As pharmacology, pharmacokinetic behavior and safety cannot be predicted simply based on a compound's chemical structure, compounds were evaluated both *in vitro* and *in vivo* for characteristics including potency, efficacy, pharmacokinetic behavior, safety, and metabolic stability, in a series of assays designed to eliminate compounds with unfavorable properties. As described herein, each of the nearly 200 compounds was first tested in several *in vitro* assays (e.g. potency, toxicology, metabolic stability), to identify a smaller set of compounds suitable for further testing in more complex *in vivo* assays (e.g. pharmacokinetic profile, efficacy, toxicology). This screening process identified a candidate pharmaceutical agent for the treatment of PKD.

### Certain Compounds of the Invention

Provided herein are compounds for use as defined in the claims, comprising a modified oligonucleotide consisting of 9 linked nucleosides, wherein the modified oligonucleotide has the following nucleoside pattern in the 5' to 3' orientation:
N_{S}N_{S}N_{M}N_{F}N_{F}N_{F}N_{M}N_{S}N_{S}
wherein nucleosides followed by subscript "M" are 2'-O-methyl nucleosides, nucleosides followed by subscript "F" are 2'-fluoro nucleosides, nucleosides followed by subscript "S" are S-cEt nucleosides, and all linkages are phosphorothioate linkages; and wherein the nucleobase sequence of the modified oligonucleotide is 5'-AGCACUUUG-3', wherein each cytosine is either a non-methylated cytosine or a 5-methylcytosine; or a pharmaceutically acceptable salt thereof. In certain embodiments, each cytosine is a non-methylated cytosine.

Provided herein is a compound comprising or consisting of the modified oligonucleotide named RG4326 for use in treating polycystic kidney disease as recited in the claims, wherein the structure of the modified oligonucleotide is:

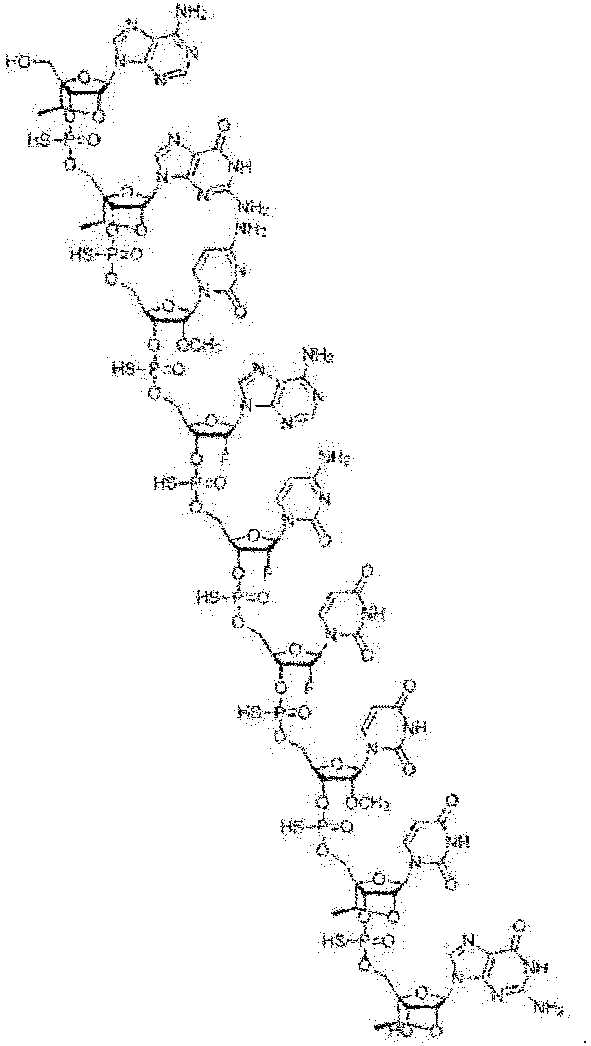

Provided herein are also pharmaceutically acceptable salts of compounds comprising or consisting of the modified oligonucleotide RG4326 for use in treating polycystic kidney disease as recited in the claims. Thus, in some embodiments, a compound comprising or consisting of the modified oligonucleotide for use as recited in the claims has the structure: 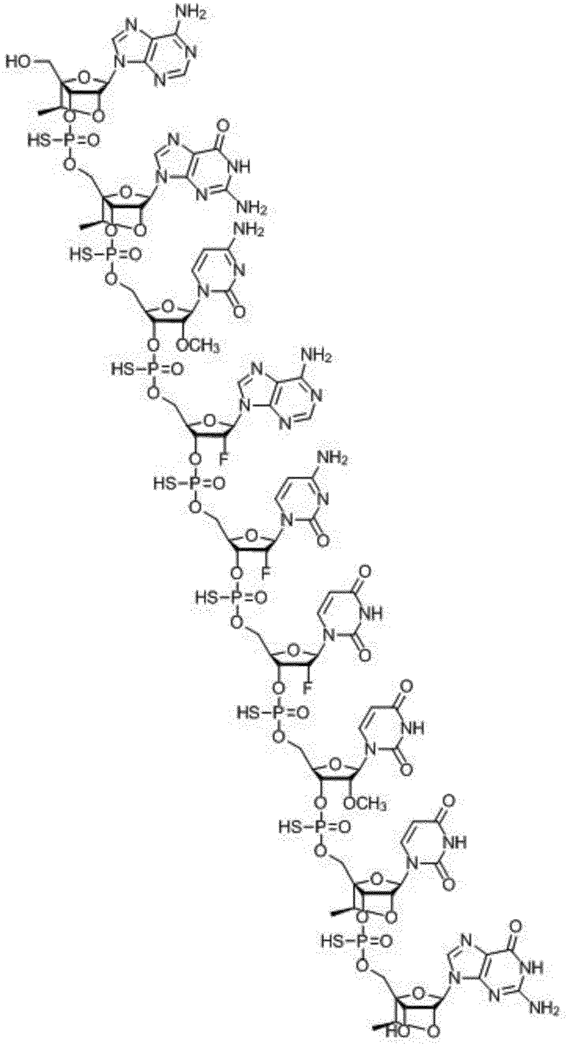 or a pharmaceutically acceptable salt thereof. A nonlimiting exemplary pharmaceutically acceptable salt of RG4326 for use as recited in the claims has the structure:

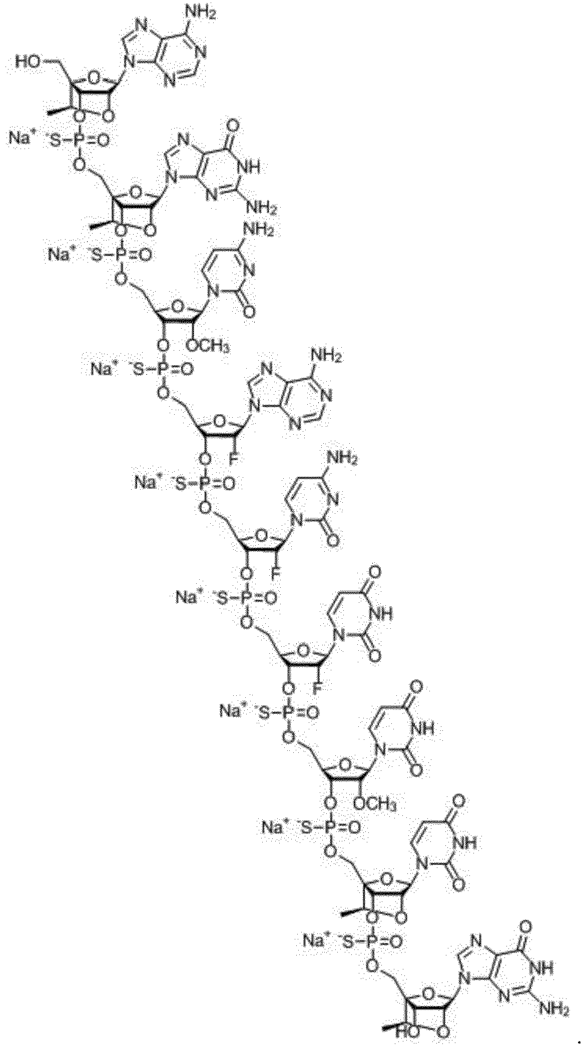

In some embodiments, a pharmaceutically acceptable salt of a modified oligonucleotide for use as recited in the claims comprises fewer cationic counterions (such as Na⁺) than there are phosphorothioate and/or phosphodiester linkages per molecule (i.e., some phosphorothioate and/or phosphodiester linkages are protonated). In some embodiments, a pharmaceutically acceptable salt of RG4326 for use as recited in the claims comprises fewer than 8 cationic counterions (such as Na⁺) per molecule of RG4326. That is, in some embodiments, a pharmaceutically acceptable salt of RG4326 for use as recited in the claims may comprise, on average, 1, 2, 3, 4, 5, 6, or 7 cationic counterions per molecule of RG4326, with the remaining phosphorothioate groups being protonated.

### Certain Uses of the Invention

Provided herein are compounds and pharmaceutical compositions for use in methods for the treatment of polycystic kidney disease (PKD) as defined in the claims, wherein the methods comprise administering the compounds or pharmaceutical compositions provided herein to a subject in need thereof, and wherein the compounds or pharmaceutical compositions comprise a nucleobase sequence complementary to the miR-17 seed sequence. In certain embodiments, the polycystic kidney disease is selected from autosomal dominant polycystic kidney disease (ADPKD), autosomal recessive polycystic kidney disease (ARPKD), and nephronophthisis (NPHP). In certain embodiments, the polycystic kidney disease is selected from autosomal dominant polycystic kidney disease (ADPKD) and autosomal recessive polycystic kidney disease (ARPKD).

In certain embodiments, the subject has a disorder that is characterized by multiple non-renal indicators, and also by polycystic kidney disease. Such disorders include, for example, Joubert syndrome and related disorders (JSRD), Meckel syndrome (MKS), or Bardet-Biedl syndrome (BBS). Accordingly, provided herein are compounds and pharmaceutical compositions for use in methods for the treatment of polycystic kidney disease (PKD) as defined in the claims, the methods comprising administering to a subject a compound or pharmaceutical composition for use as provided herein, which comprises a nucleobase sequence complementary to the miR-17 seed sequence, wherein the subject has Joubert syndrome and related disorders (JSRD), Meckel syndrome (MKS), or Bardet-Biedl syndrome (BBS). Provided herein are compounds and pharmaceutical compositions for use in methods for the treatment of polycystic kidney disease (PKD) as defined in the claims, wherein the methods comprise administering the compounds or pharmaceutical compositions for use provided herein, wherein the compounds or pharmaceutical compositions comprise a nucleobase sequence complementary to the miR-17 seed sequence, and wherein the subject is suspected of having Joubert syndrome and related disorders (JSRD), Meckel syndrome (MKS), or Bardet-Biedl syndrome (BBS).

In certain embodiments, the polycystic kidney disease is autosomal dominant polycystic kidney disease (ADPKD). ADPKD is caused by mutations in the *PKD1* or *PKD2* gene. ADPKD is a progressive disease in which cyst formation and renal enlargement lead to renal insufficiency and eventually end-stage renal disease in 50% of patients by age 60. ADPKD patients may require lifelong dialysis and/or kidney transplant. ADPKD is the most frequent genetic cause of kidney failure. The excessive proliferation of cysts is a hallmark pathological feature of ADPKD. In the management of PKD, the primary goal for treatment is to maintain kidney function and prevent the onset of end-stage renal disease (ESRD), which in turn improves life expectancy of subjects with PKD. Total kidney volume generally increases steadily in ADPKD patients, with increases correlating with a decline in kidney function. Provided herein are compounds and pharmaceutical compositions for use in methods for the treatment of ADPKD as defined in the claims, wherein the methods comprise administering the compounds or pharmaceutical compositions for use provided herein to a subject having or suspected of having ADPKD, and wherein the compounds or pharmaceutical compositions comprise a nucleobase sequence complementary to the miR-17 seed sequence.

In certain embodiments, the polycystic kidney disease is autosomal recessive polycystic kidney disease (ARPKD). ARPKD is caused by mutations in the *PKHD1* gene, and is a cause of chronic kidney disease in children. A typical renal phenotype of ARPKD is enlarged kidneys; however, ARPKD has notable effects on other organs, particularly the liver. Patients with ARPKD progress to end-stage renal disease and require a kidney transplant as young as 15 years of age. Provided herein are compounds and pharmaceutical compositions for use in methods for the treatment of ARPKD as defined in the claims, wherein the methods comprise administering the compounds or pharmaceutical compositions for use provided herein to a subject having or suspected of having ARPKD, and wherein the compounds or pharmaceutical compositions comprise a nucleobase sequence complementary to the miR-17 seed sequence.

In certain embodiments, the polycystic kidney disease is nephronophthisis (NPHP). Nephronophthisis is an autosomal recessive cystic kidney disease that is a frequent cause of ESRD in children. NPHP is characterized by kidneys of normal or reduced size, cysts concentrated at the corticomedullary junction, and tubulointerstitial fibrosis. Mutations in one of several NPHP genes, for example, *NPHP1,* have been identified in patients with NPHP. Provided herein are compounds and pharmaceutical compositions for use as defined in the claims, for use in methods for the treatment of NPHP, wherein the methods comprise administering the compounds or pharmaceutical compositions for use provided herein to a subject having or suspected of having NPHP, and wherein the compounds or pharmaceutical compositions comprise a nucleobase sequence complementary to the miR-17 seed sequence.

In certain embodiments, a subject having polycystic kidney disease has Joubert syndrome and related disorders (JSRD). JSRD includes a broad range of hallmark features, including brain, retinal, and skeletal abnormalities. Certain subjects with JSRD have polycystic kidney disease, in addition to hallmark features of JSRD. Accordingly, provided herein are compounds and pharmaceutical compositions for use as defined in the claims, for use in methods for the treatment of polycystic kidney disease in a subject having JSRD, wherein the methods comprise administering the compounds or pharmaceutical compositions for use provided herein to a subject having JSRD, and wherein the compounds or pharmaceutical compositions comprise a nucleobase sequence complementary to the miR-17 seed sequence. In certain embodiments, a subject is suspected of having JSRD.

In certain embodiments, a subject having polycystic kidney disease has Meckel syndrome (MKS). MKS is a disorder with severe signs and symptoms in many parts of the body, including the central nervous system, skeletal system, liver, kidney, and heart. Common features of MKS is the presence of numerous fluid-filled cysts in the kidney, and kidney enlargement. Accordingly, provided herein are compounds and pharmaceutical compositions for use as defined in the claims, for use in methods for the treatment of MKS, wherein the methods comprise administering the compounds or pharmaceutical compositions for use provided herein to a subject having MKS, and wherein the compounds or pharmaceutical compositions comprise a nucleobase sequence complementary to the miR-17 seed sequence. In certain embodiments, the subject is suspected of having MKS.

In certain embodiments, a subject having polycystic kidney disease has Bardet-Biedl syndrome (BBS). BBS is disorder affecting many parts of the body, including the eye, heart, kidney, liver and digestive system. A hallmark feature of BBS is the presence of renal cysts. Accordingly, provided herein are compounds and pharmaceutical compositions for use as defined in the claims, for use in methods for the treatment of polycystic kidney disease in a subject having BBS, wherein the methods comprise administering the compounds or pharmaceutical compositions for use provided herein to a subject having BBS, and wherein the compounds or pharmaceutical compositions comprise a nucleobase sequence complementary to the miR-17 seed sequence. In certain embodiments, the subject is suspected of having BBS.

In certain embodiments, the subject has been diagnosed as having PKD prior to administration of the compound comprising the modified oligonucleotide. Diagnosis of PKD may be achieved through evaluation of parameters including, without limitation, a subject's family history, clinical features (including without limitation hypertension, albuminuria, hematuria, and impaired GFR), kidney imaging studies (including without limitation MRI, ultrasound, and CT scan), and/or histological analysis.

In certain embodiments, diagnosis of PKD includes screening for mutations in one or more of the *PKD1* or *PKD2* genes. In certain embodiments, diagnosis of ARPKD includes screening for mutations in the *PKHP1* gene. In certain embodiments, diagnosis of NPHP includes screening for one or more mutations in one or more of the *NPHP1*, *NPHP2, NPHP3, NPHP4, NPHP5, NPHP6, NPHP7, NPHP8,* or *NPHP9* genes. In certain embodiments, diagnosis of JSRD includes screening for mutations in the *NPHP1, NPHP6, AHI1, MKS3, or RPGRIPIL* genes. In certain embodiments, diagnosis of MKS includes screening for mutations in the *NPHP6, MKS3, RPGRIPIL, NPHP3, CC2D2A, BBS2, BBS4, BBS6,* or *MKS1* genes. In certain embodiments, diagnosis of BBS includes screening for mutations in *BBS2, BBS4, BBS6, MKS1, BBS1, BBS3, BBS5, BBS7, BBS7, BBS8, BBS9, BBS10, BBS11,* or *BBS12* genes.

In certain embodiments, the subject has an increased total kidney volume. In certain embodiments, the total kidney volume is height-adjusted total kidney volume (HtTKV). In certain embodiments, the subject has hypertension. In certain embodiments, the subject has impaired kidney function. In certain embodiments, the subject is in need of improved kidney function. In certain embodiments, the subject is identified as having impaired kidney function.

In certain embodiments, levels of one or more miR-17 family members are increased in the kidney of a subject having PKD. In certain embodiments, prior to administration, a subject is determined to have an increased level of one or more miR-17 family members in the kidney. The level of a miR-17 family member may be measured from kidney biopsy material. In certain embodiments, prior to administration, a subject is determined to have an increased level of one or more miR-17 family members in the urine or blood of the subject.

In any of the embodiments provided herein, a subject may undergo certain tests to diagnose polycystic kidney disease in the subject, for example, to determine the cause of the polycystic kidney disease, to evaluate the extent of polycystic kidney disease in the subject, and/or to determine the subject's response to treatment. Such tests may assess markers of polycystic kidney disease. Certain of these tests, such as glomerular filtration rate and blood urea nitrogen level, are also indicators of kidney function. Markers of polycystic disease include, without limitation: measurement of total kidney volume in the subject; measurement of hypertension in the subject; assessment of kidney pain the in the subject; measurement of fibrosis in the subject; measurement of blood urea nitrogen level in the subject; measurement of serum creatinine level in the subject; measuring creatinine clearance in the subject; measuring albuminuria in the subject; measuring albumin:creatinine ratio in the subject; measuring glomerular filtration rate in the subject; measuring hematuria in the subject; measurement of NGAL protein in the urine of the subject; and/or measurement of KIM-1 protein in the urine of the subject. Unless indicated otherwise herein, blood urea nitrogen level, serum creatinine level, creatinine clearance, albuminuria, albumin:creatinine ratio, glomerular filtration rate, and hematuria refer to a measurement in the blood (such as whole blood or serum) of a subject.

Markers of polycystic kidney disease are determined by laboratory testing. The reference ranges for individual markers may vary from laboratory to laboratory. The variation may be due to, for example, differences in the specific assays used. Thus, the upper and lower limits of the normal distribution of the marker within a population, also known as the upper limit of normal (ULN) and lower limit of normal (LLN), respectively, may vary from laboratory to laboratory. For any particular marker, a health professional may determine which levels outside of the normal distribution are clinically relevant and/or indicative of disease. For example, a health professional may determine the glomerular filtration rate that may be indicative of a decline in the rate of kidney function in a subject with polycystic kidney disease.

In certain embodiments, administration of a compound for use provided herein results in one or more clinically beneficial outcomes. In certain embodiments, the administration improves kidney function in the subject. In certain embodiments, the administration slows the rate of decline of kidney function in the subject. In certain embodiments, the administration reduces total kidney volume in the subject. In certain embodiments, the administration slows the rate of increase in total kidney volume in the subject. In certain embodiments, the administration reduces height-adjusted total kidney volume (HtTKV). In certain embodiments, the administration slows the rate of increase in HtTKV.

In certain embodiments, the administration inhibits cyst growth in the subject. In certain embodiments, the administration slows rate of increase in cyst growth in the subject. In some embodiments, a cyst is present in the kidney of a subject. In some embodiments, a cyst is present in an organ other than the kidney, for example, the liver.

In certain embodiments, the administration alleviates kidney pain in the subject. In certain embodiments, the administration slows the increase in kidney pain in the subject. In certain embodiments, the administration delays the onset of kidney pain in the subject.

In certain embodiments, the administration reduces hypertension in the subject. In certain embodiments, the administration slows the worsening of hypertension in the subject. In certain embodiments, the administration delays the onset of hypertension in the subject.

In certain embodiments, the administration reduces fibrosis in kidney of the subject. In certain embodiments, the administration slows the worsening of fibrosis in the kidney of the subject.

In certain embodiments, the administration delays the onset of end stage renal disease in the subject. In certain embodiments, the administration delays time to dialysis for the subject. In certain embodiments, the administration delays time to renal transplant for the subject. In certain embodiments, the administration improves life expectancy of the subject.

In certain embodiments, the administration reduces albuminuria in the subject. In certain embodiments, the administration slows the worsening of albuminuria in the subject. In certain embodiments, the administration delays the onset of albuminuria in the subject. In certain embodiments, the administration reduces hematuria in the subject. In certain embodiments, the administration slows the worsening of hematuria in the subject. In certain embodiments, the administration delays the onset of hematuria in the subject. In certain embodiments, the administration reduces blood urea nitrogen level in the subject. In certain embodiments, the administration reduces serum creatinine level in the subject. In certain embodiments, the administration improves creatinine clearance in the subject. In certain embodiments, the administration reduces albumin:creatinine ratio in the subject.

In certain embodiments, the administration improves glomerular filtration rate in the subject. In certain embodiments, the administration slows the rate of decline of glomerular filtration rate in the subject. In certain embodiments, the glomerular filtration rate is an estimated glomerular filtration rate (eGFR). In certain embodiments, the glomerular filtration rate is a measured glomerular filtration rate (mGFR).

In certain embodiments, the administration reduces neutrophil gelatinase-associated lipocalin (NGAL) protein in the urine of the subject. In certain embodiments, the administration reduces kidney injury molecule-1 (KIM-1) protein in the urine of the subject.

In any of the embodiments, provided herein, a subject may be subjected to certain tests to evaluate the extent of disease in the subject. Such tests include, without limitation, measurement of total kidney volume in the subject; measurement of hypertension in the subject; measurement of kidney pain in the subject; measurement of fibrosis in the kidney of the subject; measurement of blood urea nitrogen level in the subject; measuring serum creatinine level in the subject; measuring creatinine clearance in the blood of the subject; measuring albuminuria in the subject; measuring albumin:creatinine ratio in the subject; measuring glomerular filtration rate in the subject, wherein the glomerular fitration rate is estimated or measured; measurement of neutrophil gelatinase-associated lipocalin (NGAL) protein in the urine of the subject; and/or measurement of kidney injury molecule-1 (KIM-1) protein in the urine of the subject.

In certain embodiments, a subject having polycystic kidney disease experiences a reduced quality of life. For example, a subject having polycystic kidney disease may experience kidney pain, which may reduce the subject's quality of life. In certain embodiments, the administration improves the subject's quality of life.

In any of the embodiments provided herein, the subject is a human subject. In certain embodiments, the human subject is an adult. In certain embodiments, an adult is at least 21 years of age. In certain embodiments, the human subject is a pediatric subject, i.e. the subject is less than 21 years of age. Pediatric populations may be defined by regulatory agencies. In certain embodiments, the human subject is an adolescent. In certain embodiments, an adolescent is at least 12 years of age and less than 21 years of age. In certain embodiments, the human subject is a child. In certain embodiments, a child is at least two years of age and less than 12 years of age. In certain embodiments, the human subject is an infant. In certain embodiments, and infant is at least one month of age and less than two years of age. In certain embodiments, the subject is a newborn. In certain embodiments, a newborn is less than one month of age.

The invention provides compounds and pharmaceutical compositions for use in the treatment of polycystic kidney disease, as defined in the claims. In certain embodiments, the polycystic kidney disease is autosomal dominant polycystic kidney disease. In certain embodiments, the polycystic kidney disease is autosomal recessive polycystic kidney disease. In certain embodiment, the polycystic kidney disease is nephronophthisis. In certain embodiments, the subject has Joubert syndrome and related disorders (JSRD), Meckel syndrome (MKS), or Bardet-Biedl syndrome (BBS).

### Certain Additional Therapies

Treatments for polycystic kidney disease in accordance with the claimed invention may comprise more than one therapy. As such, in certain embodiments, provided herein are compounds and pharmaceutical compositions for use in methods for treating a subject having or suspected of having polycystic kidney disease, as defined in the claims, wherein the methods comprise administering at least one therapy in addition to administering the compound or pharmaceutical composition for use provided herein, which comprises a nucleobase sequence complementary to the miR-17 seed sequence.

In certain embodiments, the at least one additional therapy comprises a pharmaceutical agent.

In certain embodiments, a pharmaceutical agent is an anti-hypertensive agent. Anti-hypertensive agents are used to control blood pressure of the subject.

In certain embodiments, a pharmaceutical agent is a vasopressin receptor 2 antagonist. In certain embodiments, a vasopressin receptor 2 antagonist is tolvaptan.

In certain embodiments, pharmaceutical agents include angiotensin II receptor blockers (ARB). In certain embodiments, an angiotensin II receptor blocker is candesartan, irbesartan, olmesartan, losartan, valsartan, telmisartan, or eprosartan.

In certain embodiments, pharmaceutical agents include angiotensin II converting enzyme (ACE) inhibitors. In certain embodiments, an ACE inhibitor is captopril, enalapril, lisinopril, benazepril, quinapril, fosinopril, or ramipril.

In certain embodiments, a pharmaceutical agent is a diuretic. In certain embodiments, a pharmaceutical agent is a calcium channel blocker.

In certain embodiments, a pharmaceutical agent is a kinase inhibitor. In certain embodiments, a kinase inhibitor is bosutinib or KD019.

In certain embodiments, a pharmaceutical agent is an adrenergic receptor antagonist.

In certain embodiments, a pharmaceutical agent is an aldosterone receptor antagonist. In certain embodiments, an aldosterone receptor antagonist is spironolactone. In certain embodiments, spironolactone is administered at a dose ranging from 10 to 35 mg daily. In certain embodiments, spironolactone is administered at a dose of 25 mg daily.

In certain embodiments, a pharmaceutical agent is a mammalian target of rapamycin (mTOR) inhibitor. In certain embodiments, an mTOR inhibitor is everolimus, rapamycin, or sirolimus.

In certain embodiments, a pharmaceutical agent is a hormone analogue. In certain embodiments, a hormone analogue is somatostatin or adrenocorticotrophic hormone.

In certain embodiments, a pharmaceutical agent is an anti-fibrotic agent. In certain embodiments, an anti-fibrotic agent is a modified oligonucleotide complementary to miR-21.

In certain embodiments, an additional therapy is dialysis. In certain embodiments, an additional therapy is kidney transplant.

In certain embodiments, pharmaceutical agents include anti-inflammatory agents. In certain embodiments, an anti-inflammatory agent is a steroidal anti-inflammatory agent. In certain embodiments, a steroid anti-inflammatory agent is a corticosteroid. In certain embodiments, a corticosteroid is prednisone. In certain embodiments, an anti-inflammatory agent is a non-steroidal anti-inflammatory drug. In certain embodiments, a non-steroidal anti-inflammatory agent is ibuprofen, a COX-I inhibitor, or a COX-2 inhibitor.

In certain embodiments, a pharmaceutical agent is a pharmaceutical agent that blocks one or more responses to fibrogenic signals.

In certain embodiments, an additional therapy may be a pharmaceutical agent that enhances the body's immune system, including low-dose cyclophosphamide, thymostimulin, vitamins and nutritional supplements (e.g., antioxidants, including vitamins A, C, E, beta-carotene, zinc, selenium, glutathione, coenzyme Q-10 and echinacea), and vaccines, e.g., the immunostimulating complex (ISCOM), which comprises a vaccine formulation that combines a multimeric presentation of antigen and an adjuvant.

In certain embodiments, the additional therapy is selected to treat or ameliorate a side effect of one or more pharmaceutical compositions for use of the present invention. Such side effects include, without limitation, injection site reactions, liver function test abnormalities, kidney function abnormalities, liver toxicity, renal toxicity, central nervous system abnormalities, and myopathies. For example, increased aminotransferase levels in serum may indicate liver toxicity or liver function abnormality. For example, increased bilirubin may indicate liver toxicity or liver function abnormality.

### Certain MicroRNA Nucleobase Sequences

The miR-17 family includes miR-17, miR-20a, miR-20b, miR-93, miR-106a, and miR-106b. Each member of the miR-17 family has a nucleobase sequence comprising the nucleobase sequence 5'-AAAGUG-3,' or the miR-17 seed sequence, which is the nucleobase sequence at positions 2 through 7 of SEQ ID NO: 1. Additionally, each member of the miR-17 family shares some nucleobase sequence identity outside the seed region. Accordingly, a modified oligonucleotide comprising a nucleobase sequence complementary to the miR-17 seed sequence may target other microRNAs of the miR-17 family, in addition to miR-17. For example, a modified oligonucleotide which has the nucleobase sequence 5'-AGCACUUUG-3' targets all members of the miR-17 family.

In the present invention, the nucleobase sequence of the modified oligonucleotide is 5'-AGCACUUUG-3'.

In the present invention, each cytosine is independently selected from a non-methylated cytosine and a 5-methylcytosine. In certain embodiments, at least one cytosine is a non-methylated cytosine. In certain embodiments, each cytosine is a non-methylated cytosine. In certain embodiments, at least one cytosine is a 5-methylcytosine. In certain embodiments, each cytosine is a 5-methyl cytosine.

A modified oligonucleotide having a number of linked nucleosides that is less than the length of the target microRNA, wherein each nucleobase of the modified oligonucleotide is complementary to a nucleobase at a corresponding position of the target microRNA, is considered to be a modified oligonucleotide having a nucleobase sequence that is fully complementary (also referred to as 100% complementary) to a region of the target microRNA sequence. For example, a modified oligonucleotide consisting of 9 linked nucleosides, where each nucleobase is complementary to a corresponding position of miR-17, is fully complementary to miR-17.

In certain embodiments, the modified oligonucleotide in accordance with the invention has a nucleobase sequence having one mismatch with respect to the nucleobase sequence of a target microRNA. In certain embodiments, the modified oligonucleotide has a nucleobase sequence having two mismatches with respect to the nucleobase sequence of a target microRNA. In certain such embodiments, the modified oligonucleotide has a nucleobase sequence having no more than two mismatches with respect to the nucleobase sequence of a target microRNA. In certain such embodiments, the mismatched nucleobases are contiguous. In certain such embodiments, the mismatched nucleobases are not contiguous.

Although the sequence listing accompanying this filing identifies each nucleobase sequence as either "RNA" or "DNA" as required, in practice, those sequences may be modified with a combination of chemical modifications specified herein. One of skill in the art will readily appreciate that in the sequence listing, such designation as "RNA" or "DNA" to describe modified oligonucleotides is somewhat arbitrary. For example, a modified oligonucleotide comprising a nucleoside comprising a 2'-O-methoxyethyl sugar moiety and a thymine base may described as a DNA residue in the sequence listing, even though the nucleoside is modified and is not a natural DNA nucleoside.

Accordingly, nucleic acid sequences provided in the sequence listing are intended to encompass nucleic acids containing any combination of natural or modified RNA and/or DNA, including, but not limited to such nucleic acids having modified nucleobases. By way of further example and without limitation, a modified oligonucleotide having the nucleobase sequence "ATCGATCG" in the sequence listing encompasses any oligonucleotide having such nucleobase sequence, whether modified or unmodified, including, but not limited to, such compounds comprising RNA bases, such as those having sequence "AUCGAUCG" and those having some DNA bases and some RNA bases such as "AUCGATCG" and oligonucleotides having other modified bases, such as "AT^{me}CGAUCG," wherein ^{me}C indicates a 5-methylcytosine.

### Certain Modifications

The oligonucleotide of the compounds and pharmaceutical compositions for use of the present invention is a modified oligonucleotide, as defined in the claims. A modified nucleobase, sugar, and/or internucleoside linkage may be selected over an unmodified form because of desirable properties such as, for example, enhanced cellular uptake, enhanced affinity for other oligonucleotides or nucleic acid targets and increased stability in the presence of nucleases.

The modified oligonucleotide of the compounds and pharmaceutical compositions for use of the present invention comprises sugar-modified nucleosides.

Nucleosides comprising bicyclic sugar moieties are referred to as bicyclic nucleosides or BNAs. Bicyclic nucleosides include, but are not limited to, (A) α-L-methyleneoxy(4'-CH₂-O-2') BNA; (B) β-D-methyleneoxy (4'-CH₂-O-2') BNA; (C) ethyleneoxy (4'-(CH₂)₂-O-2') BNA; (D) aminooxy (4'-CH₂-ON(R)-2') BNA; (E) oxyamino (4'-CH₂-N(R)-O-2') BNA; (F) methyl(methyleneoxy) (4'-CH(CH₃)-O-2') BNA (also referred to as constrained ethyl or cEt); (G) methylene-thio (4'-CH₂-S-2') BNA; (H) methylene-amino (4'-CH2-N(R)-2') BNA; (I) methyl carbocyclic (4'-CH₂-CH(CH₃)-2') BNA; (J) c-MOE (4'-CH(CH₂-OMe)-O-2') BNA and (K) propylene carbocyclic (4'-(CH₂)₃-2') BNA as depicted below. 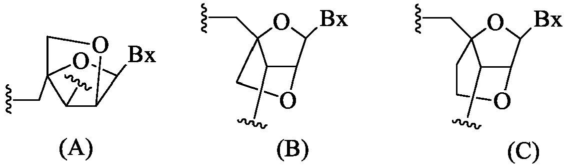 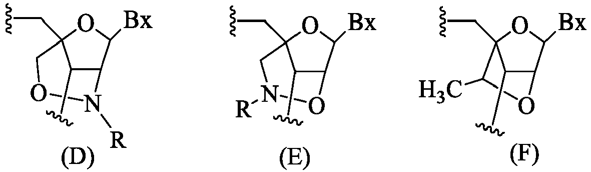 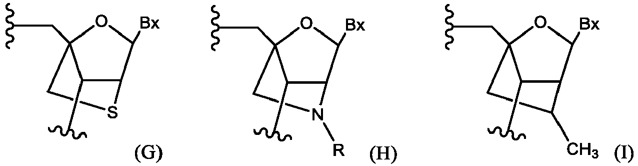 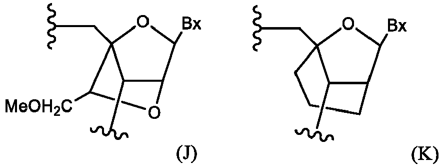 wherein Bx is a nucleobase moiety and R is, independently, H, a protecting group, or C₁-C₁₂ alkyl.

The modified oligonucleotide of the compounds and pharmaceutical compositions for use of the present invention comprises bicyclic (S-cEt) nucleosides.

The modified oligonucleotide of the compounds and pharmaceutical compositions for use of the present invention also comprises 2'-O-methyl nucleosides and 2'-fluoro nucleosides.

Each internucleoside linkage of the modified oligonucleotide of the compounds and pharmaceutical compositions for use of the present invention is a phosphorothioate internucleoside linkage.

In certain embodiments, the modified oligonucleotide of the compounds and pharmaceutical compositions for use of the present invention is conjugated to one or more moieties which enhance the activity, cellular distribution or cellular uptake of the resulting antisense oligonucleotides. In certain such embodiments, the moiety is a cholesterol moiety. In certain embodiments, the moiety is a lipid moiety. Additional moieties for conjugation include carbohydrates, peptides, antibodies or antibody fragments, phospholipids, biotin, phenazine, folate, phenanthridine, anthraquinone, acridine, fluoresceins, rhodamines, coumarins, and dyes. In certain embodiments, the carbohydrate moiety is N-acetyl-D-galactosamine (GalNac). In certain embodiments, a conjugate group is attached directly to the oligonucleotide. In certain embodiments, a conjugate group is attached to the modified oligonucleotide by a linking moiety selected from amino, azido, hydroxyl, carboxylic acid, thiol, unsaturations (e.g., double or triple bonds), 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C1-C10 alkyl, substituted or unsubstituted C2-C10 alkenyl, and substituted or unsubstituted C2-C10 alkynyl. In certain such embodiments, a substituent group is selected from hydroxyl, amino, alkoxy, azido, carboxy, benzyl, phenyl, nitro, thiol, thioalkoxy, halogen, alkyl, aryl, alkenyl and alkynyl.

In certain such embodiments, the compound for use of the present invention comprises a modified oligonucleotide having one or more stabilizing groups that are attached to one or both termini of the modified oligonucleotide to enhance properties such as, for example, nuclease stability. Included in stabilizing groups are cap structures. These terminal modifications protect a modified oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps.

### Certain Pharmaceutical Compositions

Provided herein are pharmaceutical compositions for use as defined in the claims, comprising a compound comprising or consisting of a modified oligonucleotide and a pharmaceutically acceptable diluent. In certain embodiments, the pharmaceutically acceptable diluent is an aqueous solution. In certain embodiments, the aqueous solution is a saline solution. As used herein, pharmaceutically acceptable diluents are understood to be sterile diluents. Suitable administration routes include, without limitation, intravenous and subcutaneous administration.

In certain embodiments, a pharmaceutical composition is administered in the form of a dosage unit. For example, in certain embodiments, a dosage unit is in the form of a tablet, capsule, or a bolus injection.

In certain embodiments, a pharmaceutical agent is a modified oligonucleotide which has been prepared in a suitable diluent, adjusted to pH 7.0-9.0 with acid or base during preparation, and then lyophilized under sterile conditions. The lyophilized modified oligonucleotide is subsequently reconstituted with a suitable diluent, e.g., aqueous solution, such as water or physiologically compatible buffers such as saline solution, Hanks's solution, or Ringer's solution. The reconstituted product is administered as a subcutaneous injection or as an intravenous infusion. The lyophilized drug product may be packaged in a 2 mL Type I, clear glass vial (ammonium sulfate-treated), stoppered with a bromobutyl rubber closure and sealed with an aluminum overseal.

In certain embodiments, the pharmaceutical compositions for use provided herein may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents.

In some embodiments, the pharmaceutical compositions for use provided herein may contain additional materials useful in physically formulating various dosage forms of the compositions of the present invention, such as dyes, flavoring agents, preservatives, antioxidants, opacifiers, thickening agents and stabilizers; such additional materials also include, but are not limited to, excipients such as alcohol, polyethylene glycols, gelatin, lactose, amylase, magnesium stearate, talc, silicic acid, viscous paraffin, hydroxymethylcellulose and polyvinylpyrrolidone. In various embodiments, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

Lipid moieties have been used in nucleic acid therapies in a variety of methods. In one method, the nucleic acid is introduced into preformed liposomes or lipoplexes made of mixtures of cationic lipids and neutral lipids. In another method, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, a pharmaceutical composition for use provided herein comprise a polyamine compound or a lipid moiety complexed with a nucleic acid. In certain embodiments, such preparations comprise one or more compounds each individually having a structure defined by formula (Z) or a pharmaceutically acceptable salt thereof, 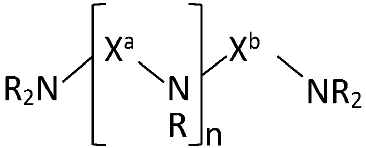 wherein each X^{a} and X^{b}, for each occurrence, is independently C₁₋₆ alkylene; n is 0, 1, 2, 3, 4, or 5; each R is independently H, wherein at least n + 2 of the R moieties in at least about 80% of the molecules of the compound of formula (Z) in the preparation are not H; m is 1, 2, 3 or 4; Y is O, NR², or S; R¹ is alkyl, alkenyl, or alkynyl; each of which is optionally substituted with one or more substituents; and R² is H, alkyl, alkenyl, or alkynyl; each of which is optionally substituted each of which is optionally substituted with one or more substituents; provided that, if n = 0, then at least n + 3 of the R moieties are not H. Such preparations are described in PCT publication WO/2008/042973. Certain additional preparations, particularly lipid preparations, are described in Akinc et al., Nature Biotechnology 26, 561 - 569 (01 May 2008).

In certain embodiments, a pharmaceutical composition for use provided herein is prepared using known techniques, including, but not limited to mixing, dissolving, granulating, dragee-making, levigating, emulsifying, encapsulating, entrapping or tableting processes.

In certain embodiments, a pharmaceutical composition for use provided herein is a solid (e.g., a powder, tablet, and/or capsule). In certain of such embodiments, a solid pharmaceutical composition comprising one or more oligonucleotides is prepared using ingredients known in the art, including, but not limited to, starches, sugars, diluents, granulating agents, lubricants, binders, and disintegrating agents.

In certain embodiments, a pharmaceutical composition for use provided herein is formulated as a depot preparation. Certain such depot preparations are typically longer acting than non-depot preparations. In certain embodiments, such preparations are administered by implantation (for example subcutaneously or intramuscularly) or by intramuscular injection. In certain embodiments, depot preparations are prepared using suitable polymeric or hydrophobic materials (for example an emulsion in an acceptable oil) or ion exchange resins, or as sparingly soluble derivatives, for example, as a sparingly soluble salt.

In certain embodiments, a pharmaceutical composition for use provided herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition for use provided herein comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition for use provided herein comprises a sustained-release system. A non-limiting example of such a sustained-release system is a semi-permeable matrix of solid hydrophobic polymers. In certain embodiments, sustained-release systems may, depending on their chemical nature, release pharmaceutical agents over a period of hours, days, weeks or months.

Certain pharmaceutical compositions for injection are presented in unit dosage form, e.g., in ampoules or in multi-dose containers.

In certain embodiments, a pharmaceutical composition for use provided herein comprises a modified oligonucleotide in a therapeutically effective amount. In certain embodiments, the therapeutically effective amount is sufficient to prevent, alleviate or ameliorate symptoms of a disease or to prolong the survival of the subject being treated.

In certain embodiments, the modified oligonucleotide may be formulated as a prodrug. In certain embodiments, upon *in vivo* administration, a prodrug is chemically converted to the biologically, pharmaceutically or therapeutically more active form of an oligonucleotide. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, a prodrug is produced by modifying a pharmaceutically active compound such that the active compound will be regenerated upon *in vivo* administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo,* those of skill in this art, once a pharmaceutically active compound is known, can design prodrugs of the compound (see, e.g., Nogrady (1985) Medicinal Chemistry A Biochemical Approach, Oxford University Press, New York, pages 388-392).

Additional administration routes include, but are not limited to, oral, rectal, transmucosal, intestinal, enteral, topical, suppository, through inhalation, intrathecal, intracardiac, intraventricular, intraperitoneal, intranasal, intraocular, intratumoral, intramuscular, and intramedullary administration. In certain embodiments, pharmaceutical intrathecals are administered to achieve local rather than systemic exposures. For example, pharmaceutical compositions may be injected directly in the area of desired effect (e.g., into the kidney).

### Certain Experimental Models

Modified oligonucleotides of the compounds and pharmaceutical compositions for use of the present invention may be used and/or tested in an experimental model. Those having skill in the art are able to select and modify the protocols for such experimental models to evaluate a pharmaceutical agent of the invention.

Generally, modified oligonucleotides are first tested in cultured cells. Suitable cell types include those that are related to the cell type to which delivery of a modified oligonucleotide is desired *in vivo.* For example, suitable cell types for the study of the methods described herein include primary or cultured cells.

The extent to which a modified oligonucleotide interferes with the activity of one or more miR-17 family members may be assessed in cultured cells. Inhibition of microRNA activity may be assessed by measuring the level of one or more of a predicted or validated microRNA-regulated transcript. An inhibition of microRNA activity may result in the increase in the miR-17 family member-regulated transcript, and/or the protein encoded by miR-17 family member-regulated transcript (i.e., the miR-17 family member-regulated transcript is de-repressed). Further, certain phenotypic outcomes may be measured.

Several animal models are available to the skilled artisan for the study of one or more miR-17 family members in models of human disease. Models of polycystic kidney disease include, but are not limited to, models with mutations and/or deletions in *Pkdl* and/or *Pkd2*; and models comprising mutations in other genes. Nonlimiting exemplary models of PKD comprising mutations and/or deletions in *Pkdl* and/or *Pkd2* include hypomorphic models, such as models comprising missense mutations in *Pkdl* and models with reduced or unstable expression of *Pkd2*; inducible conditional knockout models; and conditional knockout models. Nonlimiting exemplary PKD models comprising mutations in genes other than *Pkdl* and *Pkd2* include models with mutations in *Pkhd1*, *Nek8*, Kif3a, and/or *Nphp3.* PKD models are reviewed, *e.g.,* in Shibazaki et al., Human Mol. Genet., 2008; 17(11): 1505-1516; Happe and Peters, Nat Rev Nephrol., 2014; 10(10): 587-601; and Patel et al., PNAS, 2013; 110(26): 10765-10770.

### Certain Quantitation Assays

microRNA levels may be quantitated in cells or tissues *in vitro* or *in vivo.* Changes in microRNA levels may be measured by microarray analysis. Changes in microRNA levels may be measured by one of several commercially available PCR assays, such as the TaqMan^{®} MicroRNA Assay (Applied Biosystems).

Modulation of microRNA activity with an anti-miR or microRNA mimic may be assessed by microarray profiling of mRNAs. The sequences of the mRNAs that are modulated (either increased or decreased) by the anti-miR or microRNA mimic are searched for microRNA seed sequences, to compare modulation of mRNAs that are targets of the microRNA to modulation of mRNAs that are not targets of the microRNA. In this manner, the interaction of the anti-miR with its target microRNA, or a microRNA mimic with its targets, can be evaluated. In the case of an anti-miR, mRNAs whose expression levels are increased are screened for the mRNA sequences that comprise a seed match to the microRNA to which the anti-miR is complementary.

Modulation of microRNA activity with an anti-miR compound may be assessed by measuring the level of a messenger RNA target of the microRNA, either by measuring the level of the messenger RNA itself, or the protein transcribed therefrom. Antisense inhibition of a microRNA generally results in the increase in the level of messenger RNA and/or protein of the messenger RNA target of the microRNA, *i.e.,* anti-miR treatment results in de-repression of one or more target messenger RNAs.

### EXAMPLES

The following examples are presented in order to more fully illustrate some embodiments of the invention. They should in no way be construed, however, as limiting the broad scope of the invention.

### Example 1: The role of miR-17 in PKD

miR-17 family members of the miR-17∼92 cluster of microRNAs are upregulated in mouse models of PKD. Genetic deletion of the miR-17∼92 cluster in a mouse model of PKD reduces kidney cyst growth, improves renal function, and prolongs survival (Patel et al., PNAS, 2013; 110(26): 10765-10770). The miR-17∼92 cluster contains 6 different microRNAs, each with a distinct sequence: miR-17, miR-18a, miR-19a, miR-19-b-1 and miR-92a-1.

The miR-17∼92 cluster includes two microRNAs, miR-17 and miR-20a, that are members of the miR-17 family of microRNAs. Each member of this family shares seed sequence identity, and varying degrees of sequence identity outside the seed region. The other members of the miR-17 family are miR-20b, miR-93, miR-106a, and miR-106b. miR-20b and miR-106a reside within the miR-106a~363 cluster on the human X chromosome, and miR-93 and miR-106b reside within the miR-106b~25 cluster on human chromosome 7. The sequences of the miR-17 family members are shown in Table 1.

**Table 1: miR-17 family of microRNAs**

| **microRNA** | **SEQUENCE (5' TO 3') seed region in bold** | **SEQ ID NO:** |
|---|---|---|
| miR-17 | C**AAAGUG**CUUACAGUGCAGGUAG | 1 |
| miR-20a | U**AAAGUG**CUUAUAGUGCAGGUAG | 2 |
| miR-20b | C**AAAGUG**CUCAUAGUGCAGGUAG | 3 |
| miR-93 | C**AAAGUG**CUGUUCGUGCAGGUAG | 4 |
| miR-106a | A**AAAGUG**CUUACAGUGCAGGUAG | 5 |
| miR-106b | U**AAAGUG**CUGACAGUGCAGAU | 6 |

Previous studies using a research tool anti-miR-17 compound identified a role for miR-17 in PKD in two different models of PKD, the *Pkd2-KO* model (also known as the Pkhd1/cre;*Pkd2*^{F/F} model) and the Pcy model. The research tool modified oligonucleotide complementary to miR-17 was tested in mouse models of PKD. The anti-miR-17 compound was a fully phosphorothioated oligonucleotide 19 linked nucleosides in length (5'-CTGCACTGTAAGCACTTTG-3'; SEQ ID NO: 7), with DNA, 2'-MOE and S-cEt sugar moieties. Although the compound has mismatches with respect to other members of the miR-17 family, testing in *in vitro* assays revealed it hybridizes to and inhibits all members of the miR-17 family.

*Pkd2-KO* mice spontaneously develop polycystic kidney disease. Mice were treated with 20 mg/kg of tool anti-miR-17 compound or control oligonucleotide, or with PBS. The results demonstrated that anti-miR-17 treatment of *Pkd2-KO* mice reduced a primary treatment endpoint, kidney-to-body weight ratio, by 17%, relative to control treatment (p = 0.017). Anti-miR-17 treatment also significantly reduced BUN and expression of kidney injury mRNA biomarkers, Kim1 and Ngal, in *Pkd2-KO* mice. Finally, anti-miR-17 treatment resulted in a trend toward reduced serum creatinine level and reduced cyst index in the *Pkd2-KO* mice. These outcomes were not observed with the anti-miR-control, indicating that they are specifically due to miR-17 inhibition.

Pcy mice bearing a mutation in *Nphp3* spontaneously develop polycystic kidney disease, with a slower progression of disease than that observed in the *Pkd2-KO* mice. Mice were treated with 50 mg/kg of tool anti-miR-17 compound, or with PBS, once weekly for a total of 26 weeks. The mean ratio of kidney weight to body weight in the Pcy mice treated with anti-miR-17 was 19% lower than the mean ratio of kidney weight to body weight in the Pcy mice administered PBS only (p = 0.0003). Pcy mice treated with anti-miR-17 showed a mean 28% reduction in cyst index compared to Pcy mice administered PBS only (p = 0.008).

These data demonstrated that in two different experimental models of PKD, that miR-17 is a validated target for the treatment of PKD.

### Example 2: Compound Design and Screening

While the research tool compound showed efficacy in models of PKD, the compound was observed to be slightly proinflammatory in an *in vivo* study. Further, the research tool compound was not sufficiently efficacious for development as a pharmaceutical agent for the treatment of PKD. Accordingly, a screen was performed to identify inhibitors of one or more miR-17 family members that are sufficiently efficacious, convenient to administer, and safe for administration to subjects with PKD. An additional criterion was a sufficiently high kidney-to-liver delivery ratio, to enhance the proportion of anti-miR-17 compound that is delivered to the target organ.

Approximately 200 modified oligonucleotides comprising a nucleobase sequence complementary to the miR-17 seed sequence were designed, having varying lengths and chemical composition. The length of the compounds ranged from 9 to 20 linked nucleosides, and the compounds varied in the number, type, and placement of chemical modifications. As potency and safety cannot be predicted based on a compound's nucleobase chemical structure, compounds were evaluated both *in vitro* and *in vivo* for characteristics including potency, efficacy, pharmacokinetic behavior, viscosity, safety, and metabolic stability, in a series of assays designed to eliminate compounds with unfavorable properties. In certain assays, the tool anti-miR-17 compound was used as a benchmark to which the compounds of the library were compared. As described below, each of the nearly 200 compounds was first tested in several *in vitro* assays (e.g. potency, toxicology, metabolic stability), to identify a smaller set of compounds suitable for further testing in more complex *in vivo* assays (e.g. pharmacokinetic profile, efficacy, toxicology). The screening process was designed to identify a candidate pharmaceutical agent based on aggregated data from all assays, with an emphasis on potency, pharmacokinetic profile (e.g., delivery to the kidney), and safety characteristics.

### In Vitro and In Vivo Potency and Efficacy

*In vitro* potency was evaluated using a luciferase reporter assay. A luciferase reporter plasmid for miR-17, with two fully complementary miR-17 binding sites in tandem in the 3'-UTR of the luciferase gene. Compounds of longer lengths were selected if their maximum inhibition was greater than that of the tool anti-miR-17 compound. As shorter compounds, such as 9-mers, are typically not maximally active in the same assay conditions used for longer compounds, shorter compounds were selected based on maximum inhibition relative to appropriate control compounds. In this way, compounds that are diverse in both length and chemical composition were included in further testing.

*In vivo* potency was evaluated using the microRNA polysome shift assay (miPSA). This assay was used to determine the extent to which compounds directly engage the miR-17 target in the kidney in normal and PKD mice. The miPSA relies on the principle that active miRNAs bind to their mRNA targets in translationally active high molecular weight (HMW) polysomes, whereas the inhibited miRNAs reside in the low MW (LMW) polysomes. Treatment with anti-miR results in a shift of the microRNA from HMW polysomes to LMW polysomes. Thus, the miPSA provides a direct measurement of microRNA target engagement by a complementary anti-miR (Androsavich et al., Nucleic Acids Research, 2015, 44: e13).

Selected compounds that had passed multiple screening criteria were evaluated for efficacy in experimental models of PKD, e.g. the *Pkd2-KO* mouse model and the Pcy mouse model. Mice were treated with anti-miR-17 compound, and clinically relevant endpoints were evaluated, including the ratio of kidney weight to body weight, blood urea nitrogen level, serum creatinine levels, and kidney cyst index.

### Pharmacokinetic Properties

Metabolic stability was evaluated by incubating each anti-miR-17 compound in a mouse liver lysate. After 24 hours, the percentage of intact compound remaining is calculated. Compounds that are not stable following a 24-hour incubation are potentially not stable *in vivo.*

Pharmacokinetic properties and tissue distribution of select compounds were assessed in wild type C57BL6 mice and JCK mice (an experimental model of PKD). Compound was administered to wild type mouse at a dose of 0.3, 3, or 30 mg/kg, or to JCK mice at a dose of 3, 30, or 100 mg/kg. After seven days, mice were sacrificed. Kidney and liver tissues were collected. Concentration of anti-miR-17 compound was measured in liver and kidney. Compounds that accumulate to a greater level in kidney, relative to liver (i.e., have a higher kidney-to-liver ratio) were preferred.

A full pharmacokinetic profile for selected compounds that have passed multiple screening criteria was obtained in C57BL6 mice. In one study, mice are administered a single subcutaneous injection of anti-miR-17 compound at 30 mg/kg. In another study, mice are administered three subcutaneous injections of anti-miR-17 compound at 39 mg/kg, over a two-month period. In each study, liver and kidney samples are collected at 1 hour, 4 hours, 8 hours, 1 day, 4 days, 7 days, 14 days, 28 days, and 56 days following injections.

### Toxicology

In *in vitro* assays, the potential for toxicity was assessed using a biochemical fluorescent binding assay (FBA) and a liver or kidney slice assay. The FBA is performed by incubating a fluorescent dye with each compound, and immediately measuring fluorescence. Highly fluorescent compounds have the potential to produce toxicity *in vivo.* The liver or kidney slice assay is performed by incubating a slice of tissue from a core liver sample isolated from rat. Following a 24-hour incubation, RNA is extracted from the tissue slice, and the expression levels of 18 pro-inflammatory genes are measured. An induction in pro-inflammatory gene expression indicates a potential for pro-inflammatory effects *in vivo.*

Additional *in vivo* toxicology assessments were performed by administering to normal mice (Sv129 mice) a single subcutaneous injection of 300 mg/kg of anti-miR-17 compound. After four days, mice were sacrificed, blood was collected for serum chemistry analysis, liver and spleen were weighed, and RNA was isolated from kidney and liver tissues. The expression level of a pro-inflammatory gene, interferon-induced protein with tetratricopeptide repeats (IFIT), was measured. As an induction in IFIT expression is potentially indicative of toxicity, compounds that do not induce IFIT expression are preferred.

Throughout the screening process, certain anti-miR-17 compounds performed well in multiple assays. While no one compound was the top performer in every assay, after multiple stages of screening certain compounds exhibited particularly favorable characteristics, such as high potency and relatively high kidney-to-liver ratio. From the nearly 200 compounds that were tested in *in vitro* assays, approximately 20 met the criteria for further testing *in vivo.* These 20 compounds were eventually narrowed to five compounds, and finally to one compound, RG4326, which had the best overall profile and was selected as a candidate pharmaceutical agent. Following identification of this compound, additional studies were conducted to evaluate potency, pharmacokinetic profile, and efficacy.

RG4326 has the following sequence and chemical modification pattern: A_{S}G_{S}C_{M}A_{F}C_{F}U_{F}U_{M}U_{S}G_{S} where nucleosides followed by subscript "M" are 2'-O-methyl nucleosides, nucleosides followed by subscript "F" are 2'-fluoro nucleosides, nucleosides followed by subscript "S" are S-cEt nucleosides, each cytosine is a non-methylated cytosine and all linkages are phosphorothioate linkages. As illustrated in the following examples, this compound exhibited strong target engagement of miR-17 *in vivo,* efficacy in mouse models of PKD, and a pharmacokinetic profile that favored distribution to the kidney. Additionally, the viscosity of RG4326 was determined to be 6 cP at a concentration of approximately 150 mg/mL (in water at 20° C), thus RG4326 in solution is suitable for administration by subcutaneous injection.

### Example 3: Additional Short Anti-miR-17 Compounds

An additional nine-nucleotide compound (RG4047), in which each nucleoside is an S-cEt nucleoside, was tested in selected assays, to compare the activity, safety and pharmacokinetic profile to RG4326.

One assay employed was the luciferase assay. As noted above, short (e.g. 9 nucleotide) anti-miR-17 compounds, while they may have an advantage in *in vivo* studies, do not necessarily perform well in *in vitro* transfection assays. Accordingly, the luciferase assay transfection conditions were optimized for short anti-miR-17 compounds, so that the inhibitory activity of the compounds could be measured.

RG5124 was used as a control compound. RG5124 is 9 linked nucleosides in length, and has the same pattern of sugar modification as RG4326, but has a nucleobase sequence that is not complementary to miR-17.

The luciferase reporter plasmid for miR-17 contained a fully complementary miR-17 binding site in the 3'-UTR of the luciferase gene. HeLa cells were transfected with the microRNA mimic and its cognate luciferase reporter, followed by transfection with anti-miR-17 at doses of 0.001, 3, 10, 30, 100, and 300 nM. At the end of the 24-hour transfection period, luciferase activity was measured. As shown in Table 2, RG4047, while not as potent as RG4326, inhibited miR-17 activity in a dose dependent manner. SD indicates standard deviation.

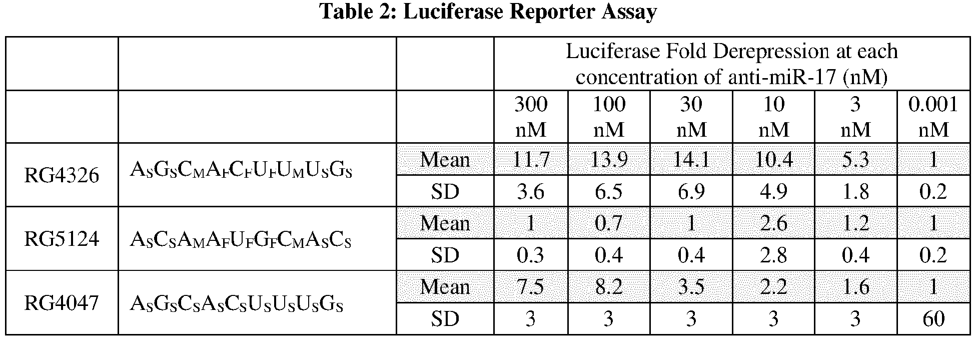

RG4047 was evaluated for potency in vivo, safety, and distribution to kidney and liver. As with the larger library screen, *in vitro* potency did not predict *in vivo* behavior. RG4047 produced a slight pro-inflammatory signal in both kidney and liver, was a less potent inhibitor of miR-17 than RG4326 *in vivo* in both wild type and PKD mice, and had a much lower kidney-to-liver ratio. These studies revealed that the activity and properties of RG4047 were not improved relative to RG4326.

### Example 4: RG4326 Activity in Additional In Vitro Assays

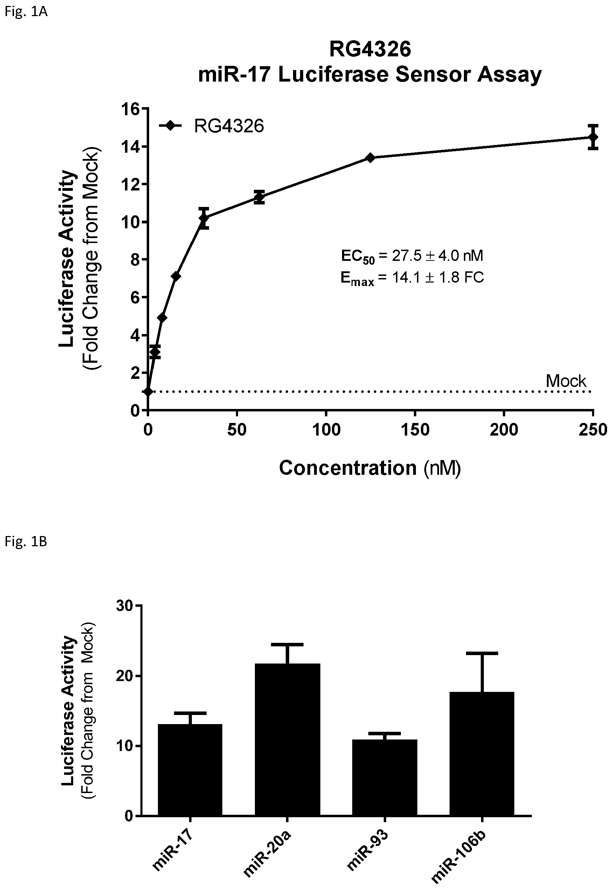

Additional *in vitro* assays were conducted to further explore the potency of RG4326. A luciferase reporter assay was used to test the ability of RG4326 to inhibit the miR-17 family members miR-17, miR-20a, miR-93, and miR-106b. A luciferase reporter plasmid for each of miR-20a, miR-93, and miR-106b was constructed, with a fully complementary microRNA binding site in the 3'-UTR of the luciferase gene. HeLa cells were transfected with the microRNA mimic and its cognate luciferase reporter, followed by transfection with anti-miR-17 at a dose of 100 nm. As shown in Table 3, each of miR-17, miR-20a, miR-93, and miR-106b was inhibited by RG4326, demonstrating that the anti-miR-17 compound inhibits multiple members of the miR-17 family. As RG4326 is 100% complementary to the other miR-17 family members not tested, miR-20b and miR-106b, it is expected to inhibit these microRNAs as well. The data in Table 3 are also shown in Figure 1A.

**Table 3: Inhibition of miR-17 family in vitro**

| | Mean Luciferase Fold Depression | SD |
|---|---|---|
| miR-17 | 13.1 | 1.6 |
| miR-20 | 21.7 | 2.8 |
| miR-93 | 10.9 | 0.9 |
| miR-106 | 17.7 | 5.5 |

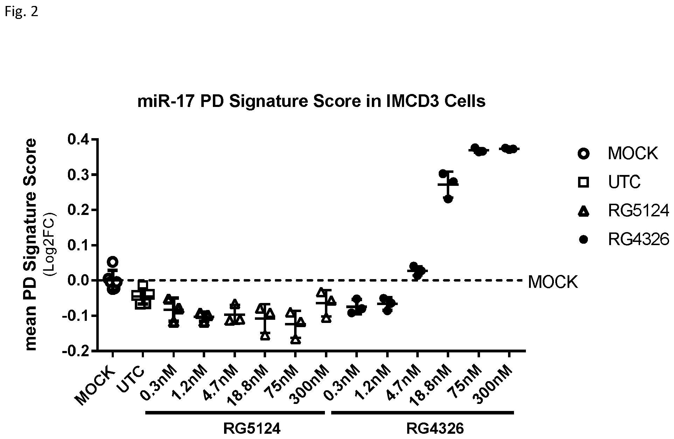

To test the ability of RG4326 to inhibit miR-17 regulation of endogenous targets, miR-17 target gene de-repression was assessed *in vitro* in several kidney cell types from normal and PKD mouse kidneys. Mouse kidney collecting duct cells (IMCD3) were treated with 0.3 nM, 1.2 nM, 4.7 nM, 18.8 nM, 75 nM, and 300 nM of RG4326 or a control oligonucleotide, RG5124. Additional control groups included untreated cells and mock-transfected cells (cell treated with transfection reagent only). After a 24-hour transfection period, cells were collected and RNA was extracted. The mRNA levels of 18 genes targeted by miR-17 were measured, and averaged to provide a pharmacodynamic signature score (PD Signature Score), represented as Log2 fold-change (Log2FC) relative to mock-transfection. As shown in Table 4, RG4326, but not control treatment, de-repressed miR-17 targets in a dose-dependent manner. The data are also shown in Figure 2B.

**Table 4: miR-17 PD Signature Score in IMCD3 cells**

| | | **Concentration of anti-miR-17 compound (nM)** | | | | | |
|---|---|---|---|---|---|---|---|
| | | **0.3** | **1.2** | **4.7** | **18.8** | **75** | **300** |
| **RG4326** | Mean Log2FC | **-0.075** | **-0.067** | **0.027** | **0.272** | **0.369** | **0.373** |
| | SD | 0.020 | 0.017 | 0.014 | 0.037 | 0.006 | 0.002 |
| **RG5124** | Mean Log2FC | -0.083 | -0.103 | -0.097 | -0.108 | -0.124 | -0.065 |
| | SD | 0.033 | 0.013 | 0.026 | 0.041 | 0.039 | 0.037 |

The ability of RG4326 to de-repress miR-17 targets was also evaluated in additional kidney cell types, derived from the kidneys of both normal and PKD mice. Cells were treated with 30 nM of RG4326 or control oligonucleotide RG5124. After a 24-hour transfection period, cells were collected and RNA was extracted. The mRNA levels of 18 genes targeted by miR-17 were measured, and averaged to provide a pharmacodynamic signature score (PD Signature Score), represented as Log2 fold-change (Log2FC) relative to mock-transfection. As shown in Table 5, RG4326, but not the control oligonucleotide, de-repressed miR-17 targets in several different healthy and diseased kidney-derived cell types. "P < 0.05" indicates a p-value of less than 0.05, as calculated by one-way ANOVA. "NS" indicates a change that is not statistically significant.

**Table 5: De-repression of miR-17 targets in kidney cell types**

| **Mouse Kidney Cell type** | **Cell Line Nomenclature** | **Mouse Kidney Origin** | **RG4326** | **RG5124** |
|---|---|---|---|---|
| | | | PD-Signature Score (Log2FC @ 30nM) | PD-Signature Score (Log2FC @ 30nM) |
| Collecting Ducts | DBA-WT | Normal | **0.40 ±0.09;** p<0.05 | 0.10 ±0.05; ns |
| Collecting Ducts | DBA-PKD | PKD | **0.52 ±0.06;** p<0.05 | -0.07 ±0.02; ns |
| Collecting Ducts | IMCD3 | Normal | **0.57 ±0.06;** p<0.05 | -0.03 ±0.05; ns |
| Collecting Ducts | M1 | Normal | **0.18 ±0.18;** p<0.05 | -0.08 ±0.02; ns |
| Distal Tubules | MDCT | Normal | **0.10 ±0.01;** p<0.05 | 0.03 ±0.01; ns |
| Proximal Tubules | LTL-WT | Normal | **0.35 ±0.02;** p<0.05 | -0.04 ±0.02; ns |
| Proximal Tubules | LTL-PKD | PKD | **0.39 ±0.01;** p<0.05 | -0.03 ±0.04; ns |

### Example 5: In Vivo Potency of RG4326

The microRNA polysome shift assay (miPSA), was used to identify compounds that directly engage miR-17 in the kidney in normal and PKD mice. The miPSA relies on the principle that active miRNAs bind to their mRNA targets in translationally active high molecular weight (HMW) polysomes, whereas the inhibited miRNAs reside in the low MW (LMW) polysomes. Treatment with anti-miR results in a shift of the microRNA from HMW polysomes to LMW polysomes. Thus, the miPSA provides a direct measurement of microRNA target engagement by a complementary anti-miR (Androsavich et al., Nucleic Acids Research, 2015, 44: e13).

For this experiment, the PKD model selected was the JCK model, a mouse model of slowly progressing renal cystic disease associated with the same gene that causes human nephronophthisis type 9. Renal cysts in this mouse develop in multiple regions of the nephron.

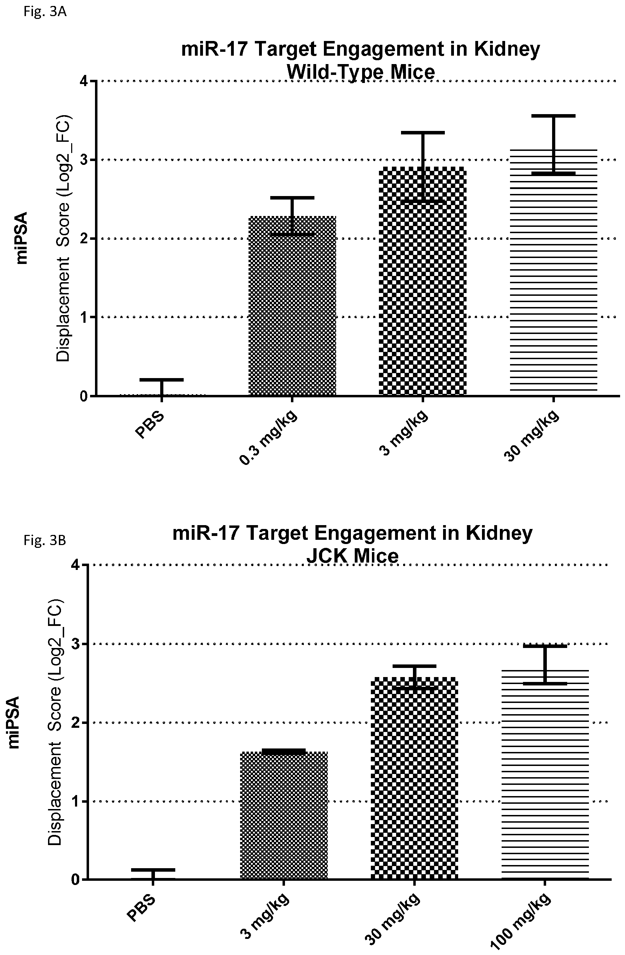

C57BL6 mice were treated with a single, subcutaneous dose of 0.3, 3, and 30 mg/kg of RG4326 or tool anti-miR-17 (described in Example 1). JCK mice were treated with a single, subcutaneous dose of 3, 30, and 100 mg/kg of RG4326 or tool anti-miR-17. PBS treatment was used as an additional control. At seven days post-treatment, mice were sacrificed, and kidney tissue was isolated for the miPSA. The calculated displacement scores, shown in Table 6, demonstrated strong target engagement by RG4326 in both normal and PKD kidneys. The displacement scores following treatment with RG4326 were greater than the displacement scores following treatment with the tool anti-miR-17 compound. The data for wild-type mice and JCK mice are also shown in Figure 3A and Figure 3B, respectively.

**Table 6: Target Engagement by RG4326 In Vivo**

| | | Normal Mice | | | | | JCK Mice | | |
|---|---|---|---|---|---|---|---|---|---|
| | | Anti-miR Dose | | | | | Anti-miR Dose | | |
| | | 0.3 mg/kg | 3 mg/kg | 30 mg/kg | | | 3 mg/kg | 30 mg/kg | 100 mg/kg |
| **RG4326** | **Mean** | **2.29** | **2.91** | **3.19** | | **Mean** | **1.63** | **2.58** | **2.73** |
| | SD | 0.52 | 0.97 | 0.81 | | SD | 0.04 | 0.32 | 0.53 |
| | | | | | | | | | |
| Tool anti-miR-17 | Mean | 1.51 | 2.05 | 2.77 | | Mean | 0.97 | 1.67 | 2.08 |
| | SD | 0.51 | 0.79 | 0.55 | | SD | 0.21 | 0.27 | 0.46 |
| | | | | | | | | | |
| PBS | Mean | 0.03 | | | | Mean | 0.00 | | |
| | SD | 0.52 | | | | SD | 0.28 | | |

### Example 6: In Vivo Efficacy of RG4326 in Experimental Models of PKD

Two experimental models of PKD were used to evaluate efficacy. *Pkd2-KO* mice spontaneously develop polycystic kidney disease, and were used as a model of ADPKD. *See* Patel et al., PNAS, 2013; 110(26): 10765-10770. Pcy mice bearing a mutation in *Nphp3* spontaneously develop polycystic kidney disease, with a slower progression of disease than that observed in the *Pkd2-KO* mice. The Pcy model is used as a model of nephronophthisis. *See* Happe and Peters, Nat. Rev. Nephrol., 2014; 10: 587-601.

### Pkd2-KO Model

RG4326 was tested in the *Pkd2-KO* mouse model of ADPKD. This model is also referred to as the *PKD2*-KO model. Wild-type mice were used as control mice. An oligonucleotide complementary to a miRNA unrelated to miR-17 was used as a treatment control for specificity (RG5124).

On each of days 10, 11 12, and 19 of age, sex-matched littermates of mice were administered a subcutaneous injection of RG4326 at a dose of 20 mg/kg (n = 12), RG5124 at a dose of 20 mg/kg (n = 12), tool anti-miR-17 at a dose of 20 mg/kg (n = 12), or PBS (n = 12). Mice were sacrificed at 28 days of age, and kidney weight, body weight, cyst index, serum creatinine level, and blood urea nitrogen (BUN) level were measured. BUN level is a marker of kidney function. A higher BUN level correlates with poorer kidney function, thus a reduction in BUN level is an indicator of reduced kidney injury and damage and improved function. Statistical significance was calculated by one-way ANOVA with Dunnett's multiple correction.

Cyst index is a histological measurement of cystic area relative to total kidney area. For this analysis, one kidney was perfused with cold PBS and 4% (wt/vol) paraformaldehyde and then harvested. Kidneys were fixed with 4% paraformaldehyde for 2 hours and then, embedded in paraffin for sectioning. Sagittal sections of kidneys were stained with hematoxylin and eosin (H&E). All image processing steps were automated and took place in freely available and open source software: An R1 script which used functions from the EBImage Bioconductor package2 and the ImageMagick3 suite of image processing tools. Kidney H&E images in Aperio SVS format were converted to TIFF images, and the first frame was retained for image analysis. First, the total kidney section area was calculated using image segmentation. Image segmentation was similarly used to find all internal structures including kidney cyst. A filter was applied to remove all objects less than a mean radius of three pixels. The cystic index is the image area associated with cysts divided by the total kidney areas. Cystic index was separately calculated for longitudinal and transverse kidney sections for each individual animal. Combined cystic index of individual animals were compared for each treatment groups.

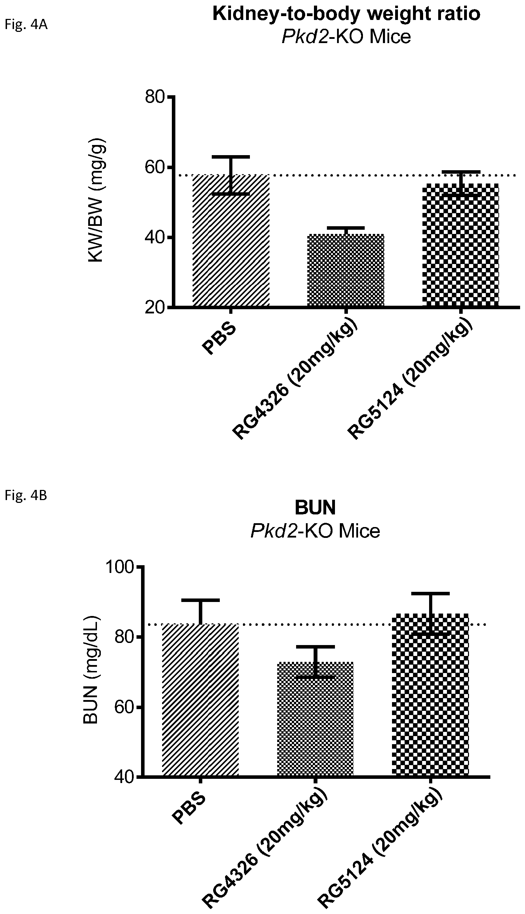

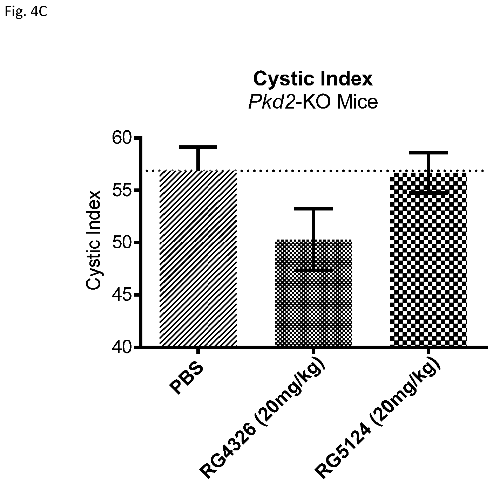

Results are shown in Table 7. The mean ratio of kidney weight to body weight (KW/BW ratio) in *Pkd2-KO* mice treated with RG4326, was 29% lower than the mean KW/BW ratio in *Pkd2-KO* mice administered PBS (p = 0.0099). *Pkd2-KO* mice treated with RG4326 showed a mean 12% reduction in cyst index compared to *Pkd2-KO* mice administered PBS, although the difference was not statistically significant. Mean BUN levels were reduced by 13% in *Pkd2-KO* mice treated with PBS, although the difference was not statistically significant. Mean serum creatinine levels in *Pkd2-KO* mice treated with RG4326 were 18% lower than in *Pkd2-KO* mice administered PBS, although the result was not statistically significant. These outcomes were not observed with the control oligonucleotide, indicating that they are specifically due to miR-17 inhibition. While a previous study demonstrated reductions in KW/BW ratio, BUN and cyst index in *Pkd2-KO* following treatment with the tool anti-miR-17 compound, no statistically significant changes were observed in this study. Treatment with the control oligonucleotide, RG5124, did not reduce kidney weight to body weight ratio, cyst index, or BUN. KW/BW ratio, BUN and cystic index are also shown in Figure 4A, Figure 4B and Figure 4C, respectively.

**Table 7: Efficacy of RG4326 in the Pkd2-KO Model of PKD**

| | | KW/BW mg/g Ratio | BUN mg/dL | Cystic Index | Serum Creatinine mg/dL |
|---|---|---|---|---|---|
| **RG4326** | **Mean** | **40.98** | **72.92** | **50.3** | **0.26** |
| | SD | 5.973 | 15.1 | 10.21 | 0.05 |
| RG5124 | Mean | 55.35 | 86.67 | 56.67 | 0.31 |
| | SD | 11.73 | 20.18 | 6.679 | 0.08 |
| PBS | Mean | 57.72 | 83.58 | 56.86 | 0.31 |
| | SD | 18.47 | 23.96 | 7.928 | 0.14 |

These results demonstrate that RG4326 treatment leads to a positive outcome in *Pkd2-KO* mice for a biological endpoint relevant to the treatment of PKD, kidney volume relative to body weight. With regard to this particular endpoint, RG4326 was more efficacious than the tool anti-miR-17 compound. RG4326 treatment resulted in a trend toward reduced BUN and reduced cyst index in the *Pkd2-KO* mice.

### Pcy Model

RG4326 was tested in the Pcy mouse model. Wild-type mice were used as control group. From four weeks of age, Pcy mice were treated once per week via subcutaneous injection with RG4326 at a dose of 25 mg/kg, tool anti-miR-17 at a dose of 25 mg/kg, control oligonucleotide RG5124 at a dose of 25 mg/kg, or PBS. Each treatment group contained 15 male mice. Three treatments were administered on 55, 56, and 57 days of age, and weekly thereafter at 6, 7, 8, 9, 10, 11, 12, 13, and 14 weeks of age. Also tested was tolvaptan, a vasopressin V2-receptor antagonist (VRA) that is prescribed to some patients with polycystic kidney disease. Mice were sacrificed at 15 weeks of age. Body weight was recorded. One kidney was extracted and weighed and the other processed for histological analysis to calculate cyst index as described for the study in the Pkhd1/cre;*Pkd2*^{F/F}. Blood urea nitrogen (BUN) level and serum creatinine level were measured. Statistical significance was calculated by one-way ANOVA with Dunnett's multiple correction.

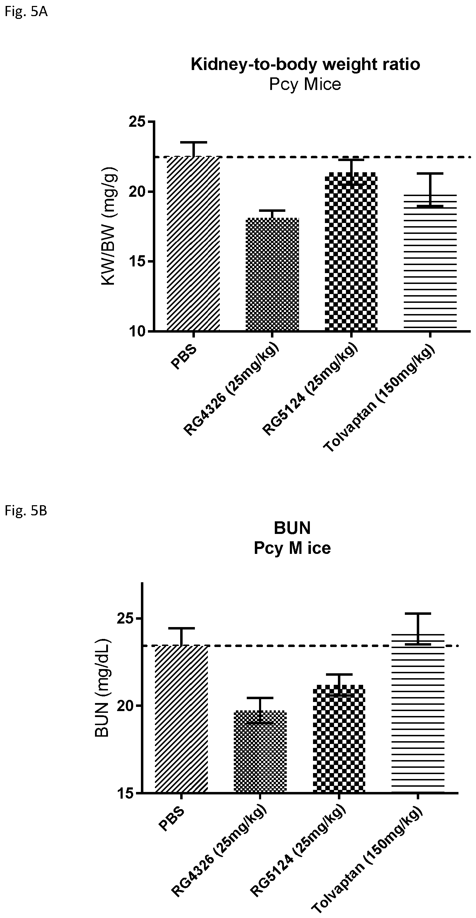

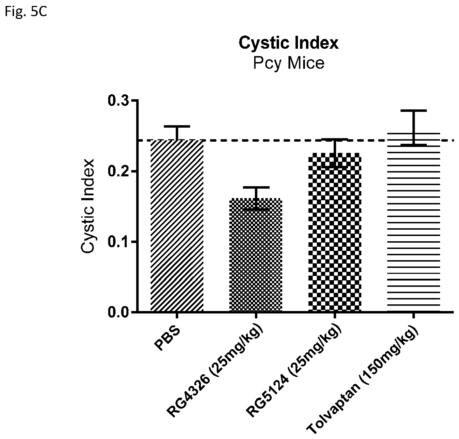

Results are shown in Table 8. Relative to the mean KW/BW ratio in the PBS-treated mice, the mean KW/BW ratio in the Pcy mice treated was 19% lower in the group treated with 25 mg/kg RG4326 (p = 0.0055). Additionally, cyst index was reduced by 34% in Pcy mice treated with RG4326 compared to Pcy mice administered PBS (p = 0.016). Treatment with RG4326 reduced BUN in Pcy mice by 16%, relative to BUN in PBS-treated Pcy mice (p = 0.0070). Treatment with the control oligonucleotide or the tool anti-miR-17 compound did not result in statistically significant reductions in KW/BW ratio, BUN or cyst index. Tolvaptan was not efficacious in this study. The data in Table 8 are also shown in Figure 5.

**Table 8: Efficacy of RG4326 in Pcy Model**

| | | KW/BW Ratio | BUN | Cystic Index |
|---|---|---|---|---|
| **RG4326** | **Mean** | **18.1** | **19.7** | **0.16** |
| | SD | 2.0 | 2.8 | 0.06 |
| RG5124 | Mean | 21.4 | 21.2 | 0.23 |
| | SD | 3.4 | 2.3 | 0.08 |
| Tolvaptan | Mean | 20.1 | 24.4 | 026 |
| | SD | 4.5 | 3.4 | 0.09 |
| PBS | Mean | 22.5 | 23.4 | 0.24 |
| | SD | 3.9 | 3.8 | 0.07 |

These data demonstrate, in an additional model of PKD, that treatment with RG4326 leads to a reduction in kidney weight, BUN and cyst index.

### Example 7: RG4326 Pharmacokinetic Assessment

Due to their reduced capacity for serum protein binding, which is a property that drives oligonucleotide distribution in the body, short oligonucleotides are not necessarily expected to have pharmacokinetic properties that make them suitable for use as drugs. RG4326 was incubated in mouse, monkey or human liver homogenate. The identity and concentration of RG4326 and metabolites was determined after a 24-hour incubation. RG4326 and metabolites were extracted using liquid-liquid extraction (LLE) and/or solid-phase extraction (SPE), which were then analyzed for identity and concentration using ion-pairing-reversed-phase high performance liquid chromatography coupled with time-of-flight mass spectrometry (IP-RP-HPLC-TOF). As shown in Table 9, despite its short length, RG4326 was found to have a particularly favorable pharmacokinetic profile, with over 95% of the parent compound RG4326 remaining intact after the 24-hour incubation.

**Table 9: In Vitro Metabolic Stability in Mouse, Monkey and Human Liver Lysate**

| ***Ex Vivo* Liver Lysate (% Analyte)** | | | | |
|---|---|---|---|---|
| **Compound** | **Sequence** | **Mouse** | **Monkey** | **Human** |
| **RG4326** | 5'-A_{S}G_{S}C_{M}A_{F}C_{F}U_{F}U_{M}U_{S}G_{S} -3' | **99.1** | **95.9** | **98.7** |
| Metabolite 1 | 5'-A_{S}G_{S}C_{M}A_{F}C_{F}-3' | **0.3** | **1.8** | **0.6** |
| Metabolite 2 | 5'-A_{S}G_{S}C_{M}A_{F}-3' | **0.6** | **2.3** | **0.7** |

Pharmacokinetic behavior was assessed by administering a single subcutaneous 30 mg/kg dose of RG4326 or tool anti-miR-17 compound to wild-type mice. At one hour, four hours, eight hours, one day, seven days, 14 days, 28 days and 56 days following the single injection, mice were sacrificed and the mean concentration of anti-miR compound in kidney and liver tissue was measured (ug/g) as described above. The area-under-curve (AUC) was calculated for kidney and liver tissue using the formula ug*h/g, where ug is the amount of oligonucleotide in the tissue, h is the timepoint of tissue collection in hours, and g is the weight of the tissue. The ratio of kidney AUC to liver AUC was determined. Kidney tissue was also processed to the miPSA, to determine target engagement for each compound in this study. PSA AUC was calculated using the formula Log2FC*h, where Log2FC is the displacement value, h is the timepoint of tissue collection in hours. Potency in the kidney at day 7 was calculated using the formula Log2FC+g/ug where Log2FC is the displacement value as determined by the miPSA, g is the weight of the kidney tissue, and ug is the amount of anti-miR in the kidney tissue at day seven.

As shown in Table 10, the ratio of kidney AUC to liver AUC for RG4326 is greater than for the tool anti-miR-17 compound. Strikingly, although the kidney AUC is lower for RG4326 than for the tool anti-miR-17 compound, the potency as determined by miPSA is substantially greater. Thus, RG4326 exhibits greater potency at lower concentrations in the kidney, the primary target tissue for PKD.

**Table 10: Pharmacokinetic Profile of RG4326**

| In Vivo Profile after single dose @ 30 mg/kg | | Tool Anti-miR-17 | **RG4326** |
|---|---|---|---|
| Pharmacokinetics | Kidney AUC (ug*h/g; one hour to 56 days) | 20711 | **5347** |
| | Liver AUC (ug*h/g; one hour to 56 days) | 20275 | **1206** |
| | K_{AUC}/L_{AUC} Ratio | 1.0 | **4.4** |
| miR-17 Inhibition | miPSA Kidney AUC (Log2FC*h; 8 hours to 7 days) | 296 | **463** |
| Potency | Kidney D7 (Log2FC*g/ug) | 0.047 | **0.351** |

The pharmacokinetic behavior of RG4326 was further characterized in wild type (C57B16) mice and PKD (JCK) mice. Groups of 5 mice each received three 10 mg/kg subcutaneous injections on each of three consecutive days. At one, four, seven, 14 and 21 days after the third and final injection, mice were sacrificed and plasma, kidney and liver samples were collected. For measurement of RG4326, RG4326 was extracted using liquid-liquid extraction (LLE) and/or solid-phase extraction (SPE), which was then analyzed for identity and concentration using ion-pairing-reversed-phase high performance liquid chromatography coupled with time-of-flight mass spectrometry (IP-RP-HPLC-TOF).

The data are summarized in Table 11. RG4326 was observed to be stable in both plasma and tissues, with over 90% of the parent compound remaining after 21 days. The anti-miR distributes to tissues rapidly, within hours of injection, and primarily to kidney. The half-life is approximately eight days in the liver and kidney of wild type mice, approximately six days in the liver of JCK mice, and approximately 8 days in the kidney of JCK mice. In wild type mice, the ratio of kidney AUC to liver AUC was 17. In PKD mice, the ratio of kidney AUC to liver AUC was 13. These data demonstrate that the pharmacokinetic profile of RG4326 is comparable in normal and PKD mice.

**Table 11: Pharmacokinetic Profile of RG4326 in Normal and PKD Mice**

| **Mouse Model** | **Normal** | | **PKD** | |
|---|---|---|---|---|
| **Mouse Strain** | C57BL6 | | JCK | |
| **Tissue Matrix** | **Liver** | **Kidney** | **Liver** | **Kidney** |
| **% Parent** | >90% | >90% | >90% | >90% |
| **C₂₄ₕ** | 1.6 ug/g | 61.4 ug/g | 5.9 ug/g | 66.5 ug/g |
| **T** _{½} | ∼8 days | ∼8 days | ∼6 days | ∼8 days |
| **AUC** _{0-21days} | 17 ug*day/g | 282 ug*day/g | 37 ug*day/g | 497 ug*day/g |
| **K/L Ratio (AUC)** | 17 | | 13 | |
| **K/L Ratio (C₂₄ₕ)** | 38 | | 11 | |

### Example 8: RG4326 Safety Assessment

The potential for toxicity in the kidney and liver was evaluated in *in vitro, ex vivo* and *in vivo* assays.

The potential for toxicity was assessed using a biochemical fluorescent binding assay (FBA). The FBA is performed by incubating a fluorescent dye with each compound, and immediately measuring fluorescence. Results are expressed as fold change (Linear FC) relative to control-treated samples. Highly fluorescent compounds have the potential to produce toxicity *in vivo.*

*Ex vivo* assays were performed with liver or kidney tissue slices. The liver or kidney slice assay is performed by incubating a slice of tissue from a core liver or kidney sample isolated from rat. Following a 24-hour incubation, RNA is extracted from the tissue slice, and the expression levels of 18 pro-inflammatory genes, including IFIT, are measured. A log2 transformation of the fold change (Log2-FC) relative to PBS treatment was performed. An induction in pro-inflammatory gene expression indicates a potential for pro-inflammatory effects *in vivo.*

An in vivo assay was performed in normal, Sv129 mice. A single, subcutaneous dose of 300 mg/kg of RG4326 was administered. Included as control treatments were PBS, and two anti-miRs not related to miR-17, one known to be pro-inflammatory (positive control) and one that is not pro-inflammatory (negative control). Four days later, mice were sacrificed. Kidney and liver tissue was isolated for RNA extraction. The level of a gene known to be induced during an inflammatory response, IFIT, was measured and normalized to mouse GAPDH. A log2 transformation of the fold change (Log2-FC) relative to PBS treatment was performed.

**Table 11: Safety Profile of RG4326**

| | **Positive Control** | **Negative Control** | **RG4326** |
|---|---|---|---|
| **Biochemical Fluorescence Binding Assay** | 136.4 ± 14.9 | 46.3 ± 14.7 | **24.9 ± 3.1** |
| Relative Fluorescence Unit (Linear FC) | | | |
| ***Ex Vivo* Kidney Slices Assay** | 1.35 ± 0.35 | 0.39 ± 0.07 | **-0.30 ± 0.22** |
| Pro-Inflammatory Signature Score (Log2-FC) | | | |
| ***Ex Vivo* Liver Slices Assay** | 7.57 ± 0.62 | 0.54 ± 0.60 | **1.11 ± 0.32** |
| IFIT3 expression (Log2-FC) | | | |
| ***In Vivo* Acute Assay** | | | |
| Kidney IFIT expression (Log2-FC) | 1.29 ± 0.58 | 0.28 ± 0.31 | **0.34 (n=1)** |
| Liver IFIT expression (Log2-FC) | 2.24 ± 0.84 | 0.62 ± 0.54 | **0.21 ± 0.08** |

These data demonstrated that RG4326 showed favorable safety profile and low risk of pro-inflammatory liability based on multiple assays.

## Claims

1. A compound for use in a method of treating polycystic kidney disease, wherein the method comprises administering the compound to a subject in need thereof, and wherein the compound comprises a modified oligonucleotide consisting of 9 linked nucleosides, wherein the modified oligonucleotide has the following nucleoside pattern in the 5' to 3' orientation:
N_{S}N_{S}N_{M}N_{F}N_{F}N_{F}N_{M}N_{S}N_{S}
wherein nucleosides followed by subscript "M" are 2'-O-methyl nucleosides, nucleosides followed by subscript "F" are 2'-fluoro nucleosides, nucleosides followed by subscript "S" are S-cEt nucleosides, and all linkages are phosphorothioate linkages; and
wherein the nucleobase sequence of the modified oligonucleotide is 5'-AGCACUUUG-3',
wherein each cytosine is independently selected from a non-methylated cytosine and a 5-methylcytosine; or a pharmaceutically acceptable salt thereof.

2. A pharmaceutical composition for use in a method of treating polycystic kidney disease, wherein the method comprises administering the pharmaceutical composition to a subject in need thereof, and wherein the pharmaceutical composition comprises:
a) a compound comprising a modified oligonucleotide consisting of 9 linked nucleosides, wherein the modified oligonucleotide has the following nucleoside pattern in the 5' to 3' orientation:
N_{S}N_{S}N_{M}N_{F}N_{F}N_{F}N_{M}N_{S}N_{S}
wherein nucleosides followed by subscript "M" are 2'-O-methyl nucleosides, nucleosides followed by subscript "F" are 2'-fluoro nucleosides, nucleosides followed by subscript "S" are S-cEt nucleosides, and all linkages are phosphorothioate linkages; and
wherein the nucleobase sequence of the modified oligonucleotide is 5'-AGCACUUUG-3', wherein each cytosine is independently selected from a non-methylated cytosine and a 5-methylcytosine; or a pharmaceutically acceptable salt thereof; and
b) a pharmaceutically acceptable diluent.

3. The compound for use according to claim 1 or the pharmaceutical composition for use according to claim 2, wherein the compound consists of the modified oligonucleotide or a pharmaceutically acceptable salt thereof.

4. The compound for use according to claim 1 or claim 3 or the pharmaceutical composition for use according to claim 2 or claim 3, wherein the pharmaceutically acceptable salt is a sodium salt.

5. The compound for use according to claim 1 or the pharmaceutical composition for use according to claim 2, wherein the modified oligonucleotide has the structure: 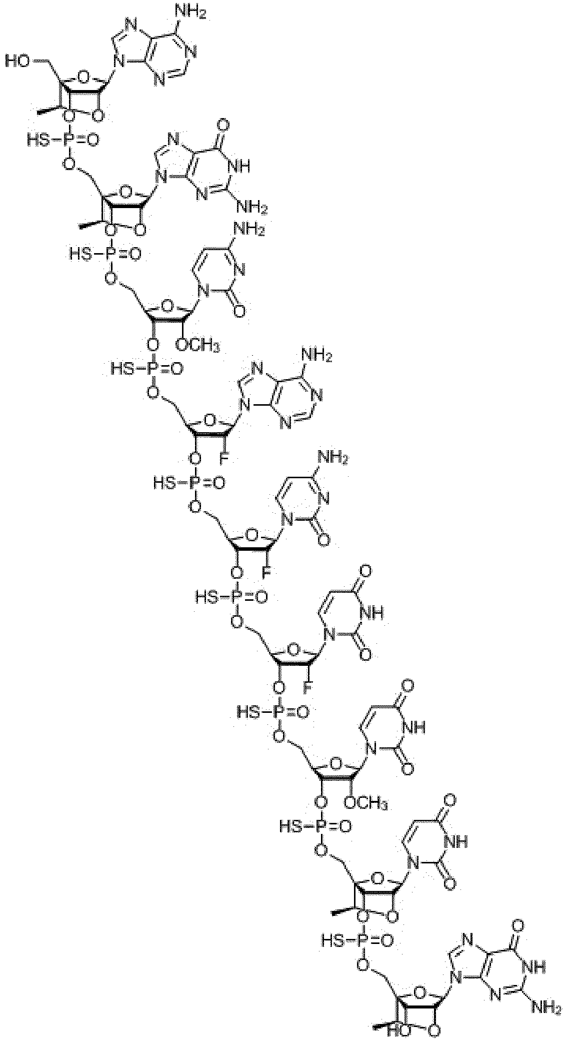 or a pharmaceutically acceptable salt thereof.

6. The compound or composition for use according to claim 5, wherein the modified oligonucleotide is a pharmaceutically acceptable salt of the structure, optionally wherein the modified oligonucleotide is a sodium salt of the structure.

7. The compound for use according to claim 1 or the pharmaceutical composition for use according to claim 2, wherein the modified oligonucleotide has the structure:

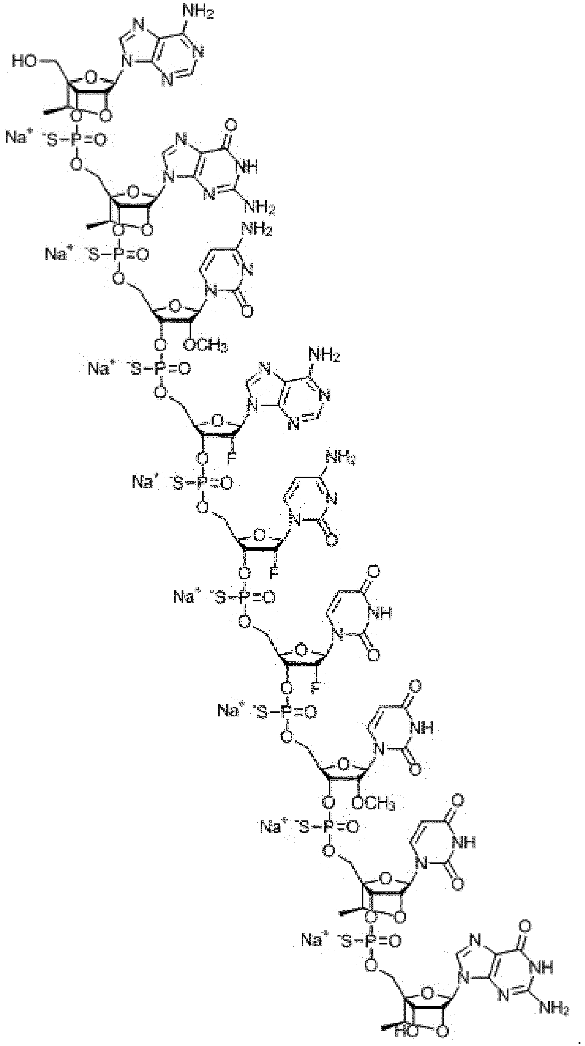

8. The pharmaceutical composition for use according to any one of claims 2 to 7, wherein the pharmaceutically acceptable diluent is a sterile aqueous solution, optionally wherein the sterile aqueous solution is a saline solution.

9. The compound for use according to any of claims 1 or 3-7, or the pharmaceutical composition for use according to any of claims 2-8, wherein the subject has been diagnosed as having polycystic kidney disease prior to administering the compound or pharmaceutical composition.

10. The compound for use according to any of claims 1, 3-7 or 9, or the pharmaceutical composition for use according to any of claims 2-9, wherein the polycystic kidney disease is autosomal recessive polycystic kidney disease, or wherein the polycystic kidney disease is autosomal dominant polycystic kidney disease.

11. The compound for use according to any of claims 1, 3-7 or 9-10, or the pharmaceutical composition for use according to any of claims 2-10, wherein the administering:
a) improves kidney function in the subject;
b) delays the worsening of kidney function in the subject;
c) reduces total kidney volume in the subject;
d) slows the increase in total kidney volume in the subject;
e) inhibits cyst growth in the subject;
f) slows the increase in cyst growth in the subject;
g) reduces kidney pain in the subject;
h) slows the increase in kidney pain in the subject;
i) delays the onset of kidney pain in the subject;
j) reduces hypertension in the subject;
k) slows the worsening of hypertension in the subject;
l) delays the onset of hypertension in the subject;
m) reduces fibrosis in the kidney of the subject;
n) slows the worsening of fibrosis in the kidney of the subject;
o) delays the onset of end stage renal disease in the subject;
p) delays time to dialysis for the subject;
q) delays time to renal transplant for the subject; and/or
r) improves life expectancy of the subject.

12. The compound for use according to any of claims 1, 3-7 or 9-11, or the pharmaceutical composition for use according to any of claims 2-11, wherein the administering:
a) reduces albuminuria in the subject;
b) slows the worsening of albuminuria in the subject;
c) delays the onset of albuminuria in the subject;
d) reduces hematuria in the subject;
e) slows the worsening of hematuria in the subject;
f) delays the onset of hematuria in the subject;
g) reduces blood urea nitrogen level in the subject;
h) reduces serum creatinine level in the subject;
i) improves creatinine clearance in the subject;
j) reduces albumin:creatinine ratio in the subject;
k) improves glomerular filtration rate in the subject;
l) slows rate of decline of glomerular filtration rate in the subject;
m) reduces neutrophil gelatinase-associated lipocalin (NGAL) protein in the urine of the subject; and/or
n) reduces kidney injury molecule-1 (KIM-1) protein in the urine of the subject.

13. The compound for use according to any of claims 1, 3-7 or 9-12, or the pharmaceutical composition for use according to any of claims 2-12, the method comprising administering at least one additional therapy selected from an angiotensin II converting enzyme (ACE) inhibitor, an angiotensin II receptor blocker (ARB), a diuretic, a calcium channel blocker, a kinase inhibitor, an adrenergic receptor antagonist, a vasodilator, a benzodiazepine, a renin inhibitor, an aldosterone receptor antagonist, an endothelin receptor blocker, an mammalian target of rapamycin (mTOR) inhibitor, a hormone analogue, a vasopressin receptor 2 antagonist, dialysis, and kidney transplant, optionally wherein:
a) the angiotensin II converting enzyme (ACE) inhibitor is selected from captopril, enalapril, lisinopril, benazepril, quinapril, fosinopril, and ramipril;
b) the angiotensin II receptor blocker (ARB) is selected from candesartan, irbesartan, olmesartan, losartan, valsartan, telmisartan, and eprosartan;
c) the vasopressin receptor 2 antagonist is tolvaptan;
d) the aldosterone receptor antagonist is spironolactone;
e) the kinase inhibitor is selected from bosutinib and KD019;
f) the mTOR inhibitor is selected from everolimus, rapamycin, and sirolimus; or
g) the hormone analogue is selected from somatostatin and adrenocorticotrophic hormone.

## Patentansprüche

1. Verbindung zur Verwendung in einem Verfahren zur Behandlung von polyzystischer Nierenerkrankung, wobei das Verfahren die Verabreichung der Verbindung an ein dessen bedürftiges Subjekt umfasst und wobei die Verbindung ein modifiziertes Oligonukleotid umfasst, das aus 9 verknüpften Nukleosiden besteht, wobei das modifizierte Oligonukleotid das folgende Nukleosidmuster in der 5' zu 3'-Orientierung aufweist:
NₛNₛN_{M}N_{F}N_{F}N_{F}N_{M}NₛNₛ
wobei es sich bei den Nukleosiden mit folgendem tiefgestellten "M" um 2'-O-Methyl-Nukleoside handelt, es sich bei den Nukleosiden mit folgendem tiefgestellten "F" um 2'-Fluor-Nukleoside handelt, es sich bei den Nukleosiden mit folgendem tiefgestellten "S" um S-cEt-Nukleoside handelt und alle Bindungen Phosphorothioatbindungen sind; und
wobei die Nukleobasensequenz des modifizierten Oligonukleotids 5'-AGCACUUUG-3' ist, wobei die Cytosine jeweils unabhängig voneinander aus einem nichtmethylierten Cytosin und einem 5-Methylcytosin ausgewählt sind; oder ein pharmazeutisch unbedenkliches Salz davon.

2. Pharmazeutische Zusammensetzung zur Verwendung in einem Verfahren zur Behandlung von polyzystischer Nierenerkrankung, wobei das Verfahren die Verabreichung der pharmazeutischen Zusammensetzung an ein dessen bedürftiges Subjekt umfasst und wobei die pharmazeutische Zusammensetzung Folgendes umfasst:
a) eine Verbindung, die ein modifiziertes Oligonukleotid umfasst, das aus 9 verknüpften Nukleosiden besteht, wobei das modifizierte Oligonukleotid das folgende Nukleosidmuster in der 5' zu 3'-Orientierung aufweist:
N_{S}N_{S}N_{M}N_{F}N_{F}N_{F}N_{M}N_{S}N_{S}
wobei es sich bei den Nukleosiden mit folgendem tiefgestellten "M" um 2'-O-Methyl-Nukleoside handelt, es sich bei den Nukleosiden mit folgendem tiefgestellten "F" um 2'-Fluor-Nukleoside handelt, es sich bei den Nukleosiden mit folgendem tiefgestellten "S" um S-cEt-Nukleoside handelt und alle Bindungen Phosphorothioatbindungen sind; und wobei die Nukleobasensequenz des modifizierten Oligonukleotids 5'-AGCACUUUG-3' ist, wobei die Cytosine jeweils unabhängig voneinander aus einem nichtmethylierten Cytosin und einem 5-Methylcytosin ausgewählt sind; oder ein pharmazeutisch unbedenkliches Salz davon; und
b) ein pharmazeutisch unbedenkliches Verdünnungsmittel.

3. Verbindung zur Verwendung nach Anspruch 1 oder pharmazeutische Zusammensetzung zur Verwendung nach Anspruch 2, wobei die Verbindung aus dem modifizierten Oligonukleotid oder einem pharmazeutisch unbedenklichen Salz davon besteht.

4. Verbindung zur Verwendung nach Anspruch 1 oder Anspruch 3 oder pharmazeutische Zusammensetzung zur Verwendung nach Anspruch 2 oder Anspruch 3, wobei es sich bei dem pharmazeutisch unbedenklichen Salz um ein Natriumsalz handelt.

5. Verbindung zur Verwendung nach Anspruch 1 oder pharmazeutische Zusammensetzung zur Verwendung nach Anspruch 2, wobei das modifizierte Oligonukleotid die folgende Struktur aufweist: 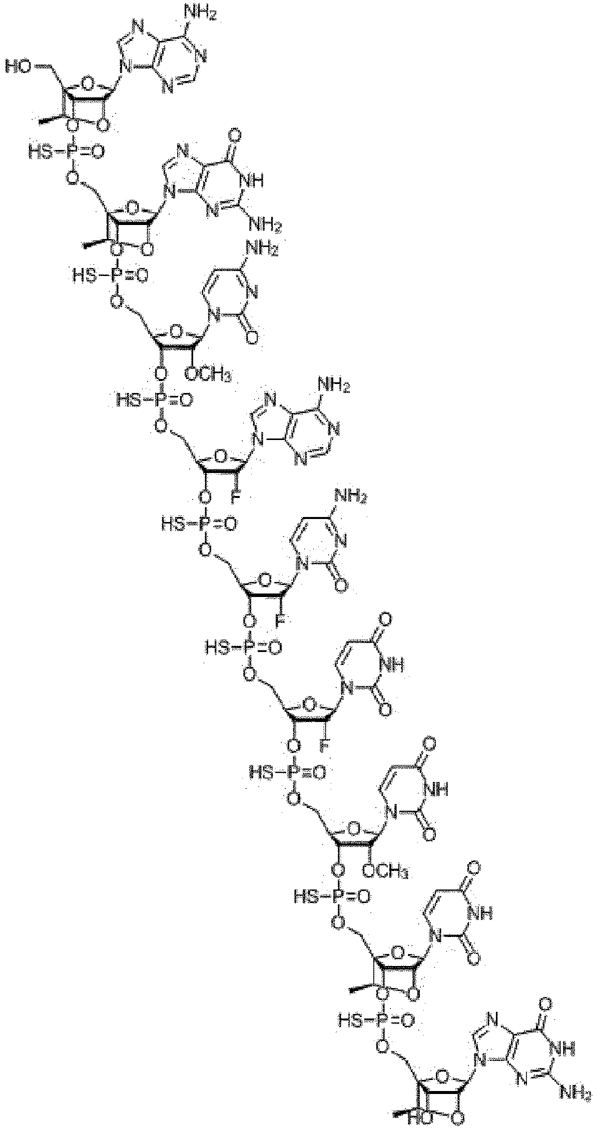 oder ein pharmazeutisch unbedenkliches Salz davon.

6. Verbindung oder Zusammensetzung zur Verwendung nach Anspruch 5, wobei es sich bei dem modifizierten Oligonukleotid um ein pharmazeutisch unbedenkliches Salz der Struktur handelt, wobei es sich bei dem modifizierten Oligonukleotid gegebenenfalls um ein Natriumsalz der Struktur handelt.

7. Verbindung zur Verwendung nach Anspruch 1 oder pharmazeutische Zusammensetzung zur Verwendung nach Anspruch 2, wobei das modifizierte Oligonukleotid die folgende Struktur aufweist:

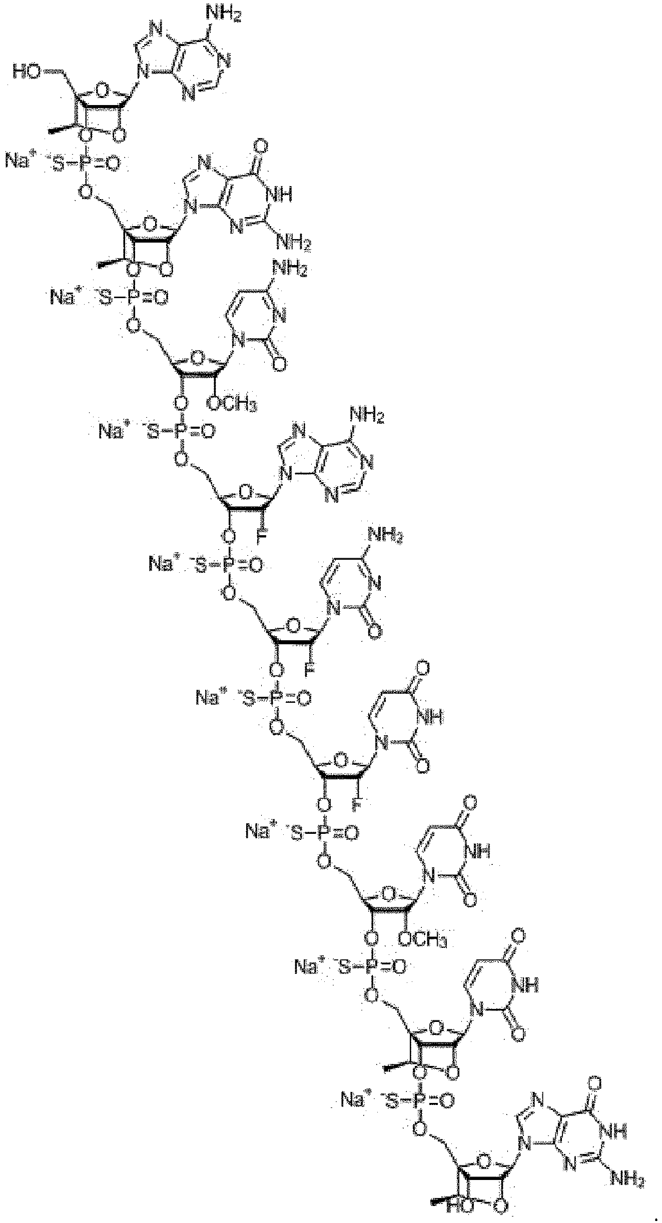

8. Pharmazeutische Zusammensetzung zur Verwendung nach einem der Ansprüche 2 bis 7, wobei es sich bei dem pharmazeutisch unbedenklichen Lösungsmittel um eine sterile wässrige Lösung handelt, wobei es sich bei der sterilen wässrigen Lösung gegebenenfalls um eine Kochsalzlösung handelt.

9. Verbindung zur Verwendung nach einem der Ansprüche 1 oder 3-7 oder pharmazeutische Zusammensetzung zur Verwendung nach einem der Ansprüche 2-8, wobei bei dem Subjekt vor der Verabreichung der Verbindung bzw. der pharmazeutischen Zusammensetzung eine polyzystische Nierenerkrankung diagnostiziert wurde.

10. Verbindung zur Verwendung nach einem der Ansprüche 1, 3-7 oder 9 oder pharmazeutische Zusammensetzung zur Verwendung nach einem der Ansprüche 2-9, wobei es sich bei der polyzystischen Nierenerkrankung um eine autosomal rezessive polyzystische Nierenerkrankung handelt oder wobei es sich bei der polyzystischen Nierenerkrankung um eine autosomal dominante polyzystische Nierenerkrankung handelt.

11. Verbindung zur Verwendung nach einem der Ansprüche 1, 3-7 oder 9-10 oder pharmazeutische Zusammensetzung zur Verwendung nach einem der Ansprüche 2-10, wobei durch die Verabreichung:
a) die Nierenfunktion des Subjekts verbessert wird;
b) die Verschlechterung der Nierenfunktion bei dem Subjekt verzögert wird;
c) das Gesamtnierenvolumens des Subjekts verringert wird;
d) die Zunahme des Gesamtnierenvolumens bei dem Subjekt verlangsamt wird;
e) das Zystenwachstum bei dem Subjekt gehemmt wird;
f) die Zunahme des Zystenwachstums bei dem Subjekt verlangsamt wird;
g) die Nierenschmerzen bei dem Subjekt verringert werden;
h) die Zunahme von Nierenschmerzen bei dem Subjekt verlangsamt wird;
i) das Auftreten von Nierenschmerzen bei dem Subjekt verzögert wird;
j) Bluthochdruck bei dem Subjekt verringert wird;
k) die Verschlimmerung des Bluthochdrucks bei dem Subjekt verlangsamt wird;
l) das Auftreten von Bluthochdruck bei dem Subjekt verzögert wird;
m) die Fibrose in der Niere des Subjekts verringert wird;
n) die Verschlimmerung der Fibrose in der Niere des Subjekts verlangsamt wird;
o) das Auftreten einer Nierenerkrankung im Endstadium bei dem Subjekt verzögert wird;
p) die Zeit bis zur Dialyse für das Subjekt verzögert wird;
q) die Zeit bis zur Nierentransplantation für das Subjekt verzögert wird; und/oder
r) die Lebenserwartung des Subjekts verbessert wird.

12. Verbindung zur Verwendung nach einem der Ansprüche 1, 3-7 oder 9-11 oder pharmazeutische Zusammensetzung zur Verwendung nach einem der Ansprüche 2-11, wobei durch die Verabreichung:
a) die Albuminurie bei dem Subjekt verringert wird;
b) die Verschlimmerung der Albuminurie bei dem Subjekt verlangsamt wird;
c) das Auftreten von Albuminurie bei dem Subjekt verzögert wird;
d) Hämaturie bei dem Subjekt verringert wird;
e) die Verschlechterung der Hämaturie bei dem Subjekt verlangsamt wird;
f) das Auftreten von Hämaturie bei dem Subjekt verzögert wird;
g) der Harnstoff-Stickstoff-Spiegel im Blut des Subjekts verringert wird;
h) der Serum-Kreatinin-Spiegel bei dem Subjekt verringert wird;
i) die Kreatinin-Clearance bei dem Subjekt verbessert wird;
j) das Albumin-Kreatinin-Verhältnis bei dem Subjekt gesenkt wird;
k) die glomeruläre Filtrationsrate bei dem Subjekt verbessert wird;
l) die Geschwindigkeit des Rückgangs der glomerulären Filtrationsrate bei dem Subjekt verlangsamt wird;
m) das Neutrophil-Gelatinase-associated-Lipocalin(NGAL)-Protein im Urin des Subjekts verringert wird; und/oder
n) das Kidney-Injury-Molecule-1(KIM-1)-Protein im Urin des Subjekts verringert wird.

13. Verbindung zur Verwendung nach einem der Ansprüche 1, 3-7 oder 9-12 oder pharmazeutische Zusammensetzung zur Verwendung nach einem der Ansprüche 2-12, wobei das Verfahren die Verabreichung mindestens einer zusätzlichen Therapie ausgewählt aus einem Angiotensin-II-Converting-Enzym-Inhibitor (ACE-Inhibitor), einem Angiotensin-II-Rezeptorblocker (ARB), einem Diuretikum, einem Calciumkanalblocker, einem Kinasehemmer, einem Adrenozeptorantagonisten, einem Vasodilatator, einem Benzodiazepin, einem Renininhibitor, einem Aldosteronrezeptorantagonisten, einem Endothelinrezeptorblocker, einem Inhibitor des Mammalian Target of Rapamycin (mTOR), einem Hormonanalogon, einem Vasopressinrezeptor-2-Antagonisten, Dialyse und Nierentransplantation umfasst, wobei gegebenenfalls:
a) der Angiotensin-II-Converting-Enzym-Inhibitor (ACE-Inhibitor) aus Captopril, Enalapril, Lisinopril, Benazepril, Quinapril, Fosinopril und Ramipril ausgewählt ist;
b) der Angiotensin-II-Rezeptorblocker (ARB) aus Candesartan, Irbesartan, Olmesartan, Losartan, Valsartan, Telmisartan und Eprosartan ausgewählt ist;
c) es sich bei dem Vasopressinrezeptor-2-Antagonisten um Tolvaptan handelt;
d) es sich bei dem Aldosteronrezeptorantagonisten um Spironolacton handelt;
e) der Kinasehemmer aus Bosutinib und KD019 ausgewählt ist;
f) der mTOR-Inhibitor aus Everolimus, Rapamycin und Sirolimus ausgewählt ist; oder
g) das Hormonanalogon aus Somatostatin und adrenokortikotropem Hormon ausgewählt ist.

## Revendications

1. Composé pour une utilisation dans un procédé de traitement d'une maladie rénale polykystique, le procédé comprenant une administration du composé à un sujet qui en a besoin, et le composé comprenant un oligonucléotide modifié constitué de 9 nucléosides liés, l'oligonucléotide modifié possédant le motif de nucléoside suivant dans la direction 5' à 3' :
N_{S}N_{S}N_{M}N_{F}N_{F}N_{F}N_{M}N_{S}N_{S}
les nucléosides suivis par l'indice « M » étant des 2'-O-méthyl-nucléosides, les nucléosides suivis par l'indice « F » étant des 2'-fluoro-nucléosides, les nucléosides suivis par l'indice « S » étant des S-cEt-nucléosides, et toutes les liaisons étant des liaisons phosphorothioate ; et
la séquence de nucléobases de l'oligonucléotide modifié étant 5'-AGCACUUUG-3', chaque cytosine étant indépendamment choisie parmi une cytosine non méthylée et une 5-méthylcytosine ; ou un sel pharmaceutiquement acceptable correspondant.

2. Composition pharmaceutique pour une utilisation dans un procédé de traitement d'une maladie rénale polykystique, le procédé comprenant une administration de la composition pharmaceutique à un sujet qui en a besoin, et la composition pharmaceutique comprenant :
a) un composé comprenant un oligonucléotide modifié constitué de 9 nucléosides liés, l'oligonucléotide modifié possédant le motif de nucléoside suivant dans la direction 5' à 3' :
N_{S}N_{S}N_{M}N_{F}N_{F}N_{F}N_{M}N_{S}N_{S}
les nucléosides suivis par l'indice « M » étant des 2'-O-méthyl-nucléosides, les nucléosides suivis par l'indice « F » étant des 2'-fluoro-nucléosides, les nucléosides suivis par l'indice « S » étant des S-cEt-nucléosides, et toutes les liaisons étant des liaisons phosphorothioate ; et la séquence de nucléobases de l'oligonucléotide modifié étant 5'-AGCACUUUG-3', chaque cytosine étant indépendamment choisie parmi une cytosine non méthylée et une 5-méthylcytosine ; ou un sel pharmaceutiquement acceptable correspondant ; et
b) un diluant pharmaceutiquement acceptable.

3. Composé pour une utilisation selon la revendication 1 ou composition pharmaceutique pour une utilisation selon la revendication 2, le composé étant constitué de l'oligonucléotide modifié ou d'un sel pharmaceutiquement acceptable correspondant.

4. Composé pour une utilisation selon la revendication 1 ou la revendication 3 ou composition pharmaceutique pour une utilisation selon la revendication 2 ou la revendication 3, le sel pharmaceutiquement acceptable étant un sel de sodium.

5. Composé pour une utilisation selon la revendication 1 ou composition pharmaceutique pour une utilisation selon la revendication 2, l'oligonucléotide modifié possédant la structure : 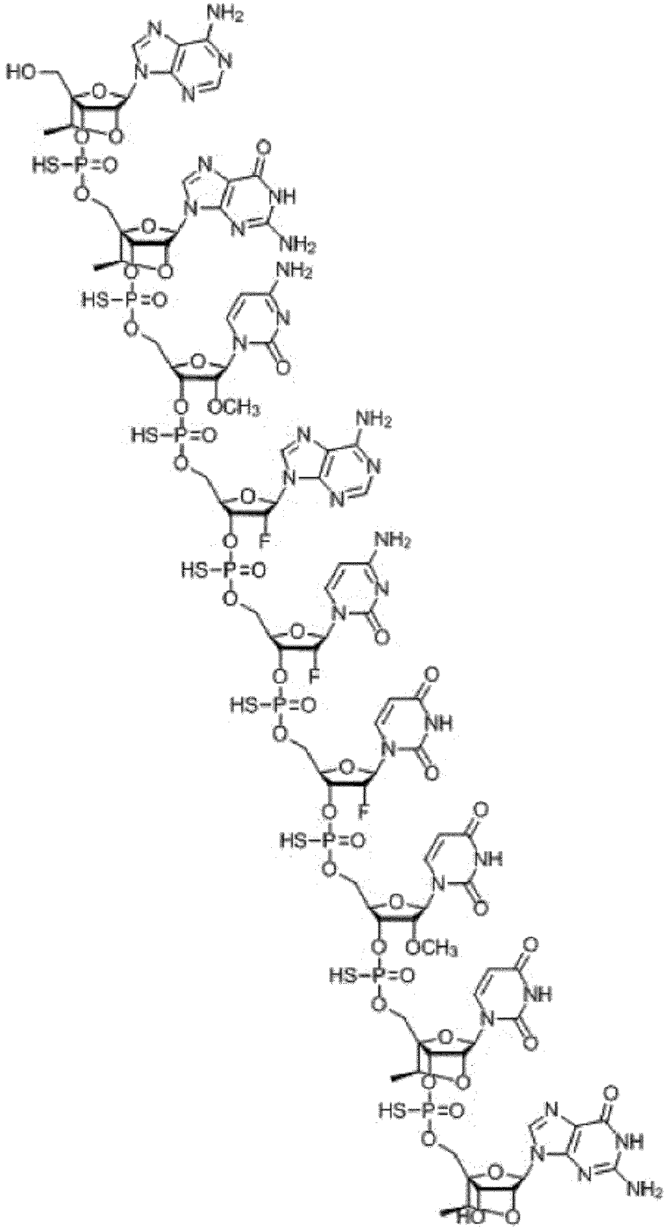 ou un sel pharmaceutiquement acceptable correspondant.

6. Composé ou composition pour une utilisation selon la revendication 5, l'oligonucléotide modifié étant un sel pharmaceutiquement acceptable de la structure, éventuellement, l'oligonucléotide modifié étant un sel de sodium de la structure.

7. Composé pour une utilisation selon la revendication 1 ou composition pharmaceutique pour une utilisation selon la revendication 2, l'oligonucléotide modifié possédant la structure :

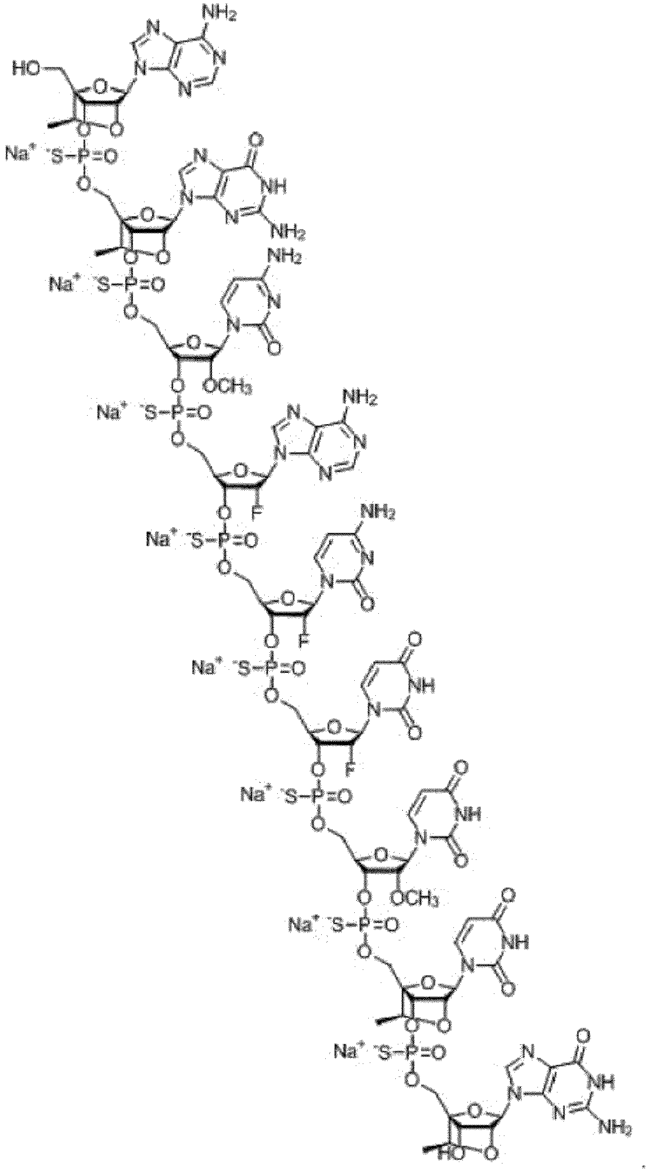

8. Composition pharmaceutique pour une utilisation selon l'une quelconque des revendications 2 à 7, le diluant pharmaceutiquement acceptable étant une solution aqueuse stérile, éventuellement, la solution aqueuse stérile étant une solution saline.

9. Composé pour une utilisation selon l'une quelconque des revendications 1 ou 3 à 7, ou composition pharmaceutique pour une utilisation selon l'une quelconque des revendications 2 à 8, le sujet ayant été diagnostiqué comme étant atteint d'une maladie rénale polykystique avant une administration du composé ou de la composition pharmaceutique.

10. Composé pour une utilisation selon l'une quelconque des revendications 1 ou 3 à 7 ou 9, ou composition pharmaceutique pour une utilisation selon l'une quelconque des revendications 2 à 9, la maladie rénale polykystique étant une maladie rénale polykystique récessive autosomale, ou la maladie rénale polykystique étant une maladie rénale polykystique dominante autosomale.

11. Composé pour une utilisation selon l'une quelconque des revendications 1, 3 à 7 ou 9 à 10, ou composition pharmaceutique pour une utilisation selon l'une quelconque des revendications 2 à 10, où l'administration :
a) améliore la fonction rénale chez le sujet ;
b) retarde l'aggravation de la fonction rénale chez le sujet ;
c) réduit le volume total des reins chez le sujet ;
d) ralentit l'augmentation du volume total des reins chez le sujet ;
e) inhibe la croissance des kystes chez le sujet ;
f) ralentit l'augmentation de la croissance des kystes chez le sujet ;
g) réduit la douleur rénale chez le sujet ;
h) ralentit l'augmentation de la douleur rénale chez le sujet ;
i) retarde l'apparition de la douleur rénale chez le sujet ;
j) réduit l'hypertension chez le sujet ;
k) ralentit l'aggravation de l'hypertension chez le sujet ;
l) retarde l'apparition de l'hypertension chez le sujet ;
m) réduit la fibrose dans le rein du sujet ;
n) ralentit l'aggravation de la fibrose dans le rein du sujet ;
o) retarde l'apparition de l'insuffisance rénale terminale chez le sujet ;
p) retarde le moment de la dialyse chez le sujet ;
q) retarde le moment de la transplantation rénale pour le sujet ; et/ou
r) améliore l'espérance de vie du sujet.

12. Composé pour une utilisation selon l'une quelconque des revendications 1, 3 à 7 ou 9 à 11, ou composition pharmaceutique pour une utilisation selon l'une quelconque des revendications 2 à 11, où l'administration :
a) réduit l'albuminurie chez le sujet ;
b) ralentit l'aggravation de l'albuminurie chez le sujet ;
c) retarde l'apparition de l'albuminurie chez le sujet ;
d) réduit l'hématurie chez le sujet ;
e) ralentit l'aggravation de l'hématurie chez le sujet ;
f) retarde l'apparition de l'hématurie chez le sujet ;
g) réduit le taux d'azote uréique sanguin chez le sujet ;
h) réduit le taux de créatinine sérique chez le sujet ;
i) améliore la clairance de la créatinine chez le sujet ;
j) réduit le rapport albumine: créatinine chez le sujet ;
k) améliore le taux de filtration glomérulaire chez le sujet ;
l) ralentit la vitesse de déclin du taux de filtration glomérulaire chez le sujet ;
m) réduit la protéine lipocaline associée à la gélatinase des neutrophiles (NGAL) dans l'urine du sujet ; et/ou
n) réduit la protéine molécule 1 de lésion rénale (KIM-1) dans l'urine du sujet.

13. Composé pour une utilisation selon l'une quelconque des revendications 1, 3 à 7 ou 9 à 12, ou composition pharmaceutique pour une utilisation selon l'une quelconque des revendications 2 à 12, le procédé comprenant l'administration d'au moins une thérapie supplémentaire choisie parmi un inhibiteur de l'enzyme de conversion de l'angiotensine II (ACE), un bloqueur des récepteurs de l'angiotensine II (ARB), un diurétique, un bloqueur des canaux calciques, un inhibiteur de kinase, un antagoniste des récepteurs adrénergiques, un vasodilatateur, une benzodiazépine, un inhibiteur de la rénine, un antagoniste du récepteur de l'aldostérone, un bloqueur du récepteur de l'endothéline, un inhibiteur de la cible mammalienne de la rapamycine (mTOR), un analogue hormonal, un antagoniste du récepteur 2 de la vasopressine, une dialyse et une transplantation rénale, éventuellement où :
a) l'inhibiteur de l'enzyme de conversion de l'angiotensine II (ACE) est choisi parmi le captopril, l'énalapril, le lisinopril, le bénazépril, le quinapril, le fosinopril et le ramipril ;
b) le bloqueur des récepteurs de l'angiotensine II (ARB) est choisi parmi le candésartan, l'irbésartan, l'olmésartan, le losartan, le valsartan, le telmisartan et l'éprosartan ;
c) l'antagoniste du récepteur 2 de la vasopressine est le tolvaptan ;
d) l'antagoniste du récepteur de l'aldostérone est la spironolactone ;
e) l'inhibiteur de kinase est choisi parmi le bosutinib et le KD019 ;
f) l'inhibiteur de mTOR est choisi parmi l'évérolimus, la rapamycine et le sirolimus ; ou
g) l'analogue d'hormone est choisi parmi la somatostatine et l'hormone adrénocorticotrophique.
